# EUROPEAN PATENT SPECIFICATION

(11) **EP 2 729 802 B1**
(45) Date of publication and mention of the grant of the patent: **06.05.2015**
(21) Application number: 12748286.7
(22) Date of filing: 06.07.2012
(51) Int. Cl.: G01N 33/50, G01N 1/28

(54) **METHOD OF ANALYZING CARDIOVASCULAR DISORDERS AND USES THEREOF**
VERFAHREN ZUR ANALYSE VON HERZ-KREISLAUF-ERKRANKUNGEN UND VERWENDUNGEN DAVON
MÉTHODE D'ANALYSE DES TROUBLES CARDIO-VASCULAIRES ET SES UTILISATIONS

(30) Priority: 07.07.2011 US 201161505481 P
(43) Date of publication of application: 14.05.2014
(73) Proprietor: Scripps Health, San Diego, CA 92121 (US); Janssen Diagnostics, LLC, Raritan, NJ 08869 (US)
(72) Inventor: TOPOL, Eric, La Jolla, CA 92037 (US); DAMANI, Samir, San Diego, CA 92130 (US); CONNELLY, Mark, Doylestown, PA 18901 (US); RAO, Galla, Chandra, Princeton Junction, NJ 08550 (US)
(74) Representative: Irvine, Jonquil Claire
(86) International application number: PCT/US2012/045832
(87) International publication number: WO 2013/006828

(56) References cited:
- WO-A2-2009/143478
- US-A1- 2011 195 413
- T B WILLS ET AL: "Immunomagnetic isolation of canine circulating endothelial and endothelial progenitor cells", VETERINARY CLINICAL PATHOLOGY, vol. 38, no. 4, 2009, pages 437-442, XP55041567,
- KELLY A S ET AL: "Circulating Activated Endothelial Cells in Pediatric Obesity", JOURNAL OF PEDIATRICS, vol. 157, no. 4, 2010, pages 547-551, XP027256202, [retrieved on 2010-06-14]
- A. WOYWODT ET AL: "An improved assay for enumeration of circulating endothelial cells", ANNALS OF HEMATOLOGY, vol. 83, no. 8, 2004, pages 491-494, XP55042176,
- MUTIN M ET AL: "DIRECT EVIDENCE OF ENDOTHELIAL INJURY IN ACUTE MYOCARDIAL INFARCTION AND UNSTABLE ANGINE BY DEMONSTRATION OF CIRCULATING ENDOTHELIAL CELLS", BLOOD, vol. 93, no. 9, 1999, pages 2951-2958, XP001180171,
- C. J. BOOS ET AL: "Relationship between circulating endothelial cells and the predicted risk of cardiovascular events in acute coronary syndromes", EUROPEAN HEART JOURNAL, vol. 28, no. 9, 2007, pages 1092-1101, XP55042170,
- QUILICI J ET AL: "Circulating Endothelial Cell Count as a Diagnostic Marker for Non-ST-Elevation Acute Coronary syndromes", CIRCULATION, vol. 110, no. 12, 2004, pages 1586-1591, XP008121979, [retrieved on 2004-09-13]
- DATABASE MEDLINE [Online] US NATIONAL LIBRARY OF MEDICINE (NLM), BETHESDA, MD, US; March 2011 (2011-03), SONG TIE-NIU ET AL: "[Circulating endothelial cell injury in on-pump and off-pump coronary-artery bypass grafting].", XP002686078, Database accession no. NLM21421501 & NAN FANG YI KE DA XUE XUE BAO = JOURNAL OF SOUTHERN MEDICAL UNIVERSITY, vol. 31, no. 3, March 2011 (2011-03), pages 535-538,
- DAMANI S ET AL: "Characterization of Circulating Endothelial Cells in Acute Myocardial Infarction", SCIENCE TRANSLATIONAL MEDICINE, vol. 4, no. 126, March 2012 (2012-03), pages 139-147, XP8157357,

## Description

### BACKGROUND OF THE INVENTION

Acute myocardial infarction (MI) and ischemic stroke remain leading causes of death and disability worldwide. Each year, over 2.5 million individuals in the United States experience a new or recurrent heart attack or ischemic stroke. Currently, stable coronary artery disease (CAD) is readily diagnosed through functional stress testing and coronary angiography.

Boos et al, European Heart Journal (2007) 28, 1092-1101 discloses the relationship between circulating endothelial cells and the predicted risk of cardiovascular events in acute coronary syndromes.

### SUMMARY OF THE INVENTION

The invention is defined as follows:
1. A method of analyzing a cardiovascular disorder in an individual in need thereof, comprising:
   (a) obtaining a magnified image of a population of circulating endothelial cells (CECs) isolated from a blood sample obtained from the individual; and
   (b) comparing two or more morphological features of the population of circulating endothelial cells in the magnified image to a same morphological feature of a control or standard, wherein the morphological feature is: cellular area, nuclear area, ratio of cellular area to nuclear area, cell shape, nuclei shape, number of nuclei, number of circulating endothelial cells, or a combination thereof.
2. The method of item 1, further comprising diagnosing the individual with a cardiovascular disorder if the morphological feature(s) of the population of circulating endothelial cells (a) deviates from the control or standard from a healthy individual or population of healthy individuals, or (b) matches the control or standard from an individual or population of individuals who have or have had the cardiovascular disease.
3. The method of item 1, further comprising predicting the susceptibility of the individual for developing a cardiovascular disorder based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease.
4. The method of item 1, further comprising prescribing a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease.
5. The method of item 1, further comprising predicting the individual's response to a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease.
6. The method of any of items 2 to 5, wherein the deviation is the population of circulating endothelial cells having an average cellular area that is greater than the cellular area of CECs from a control or standard derived from a healthy individual or population of healthy individuals;
   the deviation is the population of circulating endothelial cells having an average nuclear area that is greater than the nuclear area of CECs from a control or standard derived from a healthy individual or population of healthy individuals;
   the deviation is the population of circulating endothelial cells having an average ratio of cellular area to nuclear area that is greater than the average ratio of cellular area to nuclear area of CECs from a control or standard derived from a healthy individual or population of healthy individuals;
   the deviation is the population of circulating endothelial cells having an average number of nuclei that is greater than the number of nuclei of CECs from a control or standard derived from a healthy individual or population of healthy individuals;
   the deviation is the population of circulating endothelial cells having an abnormal cellular shape as compared to the cellular shape of CECs from a control or standard derived from a healthy individual or population of healthy individuals; or
   the deviation is the population of circulating endothelial cells having an abnormal nuclear shape as compared to the nuclear shape of CECs from a control or standard derived from a healthy individual or population of healthy individuals.
7. The method of any of items 2 to 5, wherein the deviation is the population of circulating endothelial cells having an average cellular area that is at least about 30% greater than the average cellular area of CECs from a control or standard derived from a healthy individual or population of healthy individuals.
8. The method of any of items 2 to 5, wherein the deviation is the population of circulating endothelial cells having an average cellular area that is greater than about 100 µm².
9. The method of any of items 2 to 5, wherein the deviation is the population of circulating endothelial cells having an average nuclear area that is at least about 30% greater than the average nuclear area of CECs from a control or standard derived from a healthy individual or population of healthy individuals.
10. The method of any of items 2 to 5, wherein the deviation is the population of circulating endothelial cells having an average nuclear area that is greater than about 40 µm².
11. The method of any of items 2 to 5, wherein the deviation is the population of circulating endothelial cells having an average of at least two nuclei per CEC.
12. The method of any of items 1 to 11, further comprising comparing:
   (a) the concentration of the population of circulating endothelial cells isolated from the blood sample; and
   (b) the concentration of a control or standard.
13. The method of any of items 1 to 12, wherein the population of circulating endothelial cells is isolated from the blood sample by:
   (a) contacting the blood sample with anti-CD146 antibodies, wherein the antibodies are magnetically labeled or fluorescently labeled; and
   (b) isolating the cells to which the anti-CD146 antibodies specifically bind, to generate a population of CD146+ cells.
14. The method of item 13, further comprising contacting the population of CD146+ cells with fluorescently labeled anti-CD45 antibodies, anti-CD105 antibodies, anti-CD34 antibodies, anti-CD31 antibodies, anti-VEGF2 antibodies, anti-CD144 antibodies, anti-CD106 antibodies, DAPI, or a combination thereof.
15. The method of items 13 or 14, further comprising discarding a CD146+ cell that specifically binds to an anti-CD45 antibody and retaining a CD146+ cell that does not specifically bind to an anti-CD45 antibody, to generate a population of isolated circulating endothelial cells.
16. The method of any of items 1 to 15, wherein the cardiovascular disorder is selected from:
   plaque rupture, plaque erosion, ischemia of the heart, reperfusion injury to the heart, atherosclerosis, acute coronary syndrome, ST-segment elevation myocardial infarction (STEMI), non-ST-segment elevation myocardial infarction (NSTEMI), unstable angina, ischemic stroke or a combination thereof.

The following disclosed embodiments are not necessarily part of the invention. Only the disclosed embodiments that fall within the scope of the items presented above are part of the invention.

Disclosed herein, in certain embodiments, are systems for analyzing a cardiovascular disorder, comprising: (a) a population of circulating endothelial cells (CEC) isolated from a blood sample obtained from an individual, wherein the blood sample comprises cells selected from CECs and circulating non-endothelial cells; and (b) a microscope having sufficient magnifying power to visualize a morphological feature of a single CEC in the population of CECs; wherein the morphological feature is selected from: cellular area, nuclear area, ratio of cellular area to nuclear area, cell shape, nuclei shape, number of nuclei, number of circulating endothelial cells, or a combination thereof. In some embodiments, the systems further comprise electronic memory for capturing and storing a magnified image of the population of CECs or a single CEC in the population of CECs. In some embodiments, the systems further comprise a computer-processing device, optionally connected to a computer network. In some embodiments, the systems further comprise a software module executed by the computer-processing device to analyze a morphological feature of the population of circulating endothelial cells (CEC). In some embodiments, the systems further comprise a software module executed by the computer-processing device to compare the morphological feature of the population of circulating endothelial cells (CEC) to a standard or control. In some embodiments, the systems further comprise a software module executed by the computer-processing device to determine concentration of the population of circulating endothelial cells isolated from the blood sample. In some embodiments, the systems further comprise a software module executed by the computer-processing device to compare the concentration of the population of circulating endothelial cells to the concentration of control or standard. In some embodiments, the systems further comprise a machine to isolate the population of circulating endothelial cells from the blood sample. In some embodiments, the systems further comprise a label that specifically binds to circulating endothelial cells, a label that does not bind to circulating endothelial cells, or a combination thereof In some embodiments, the systems further comprise a label that specifically binds to CD146, a label that specifically binds to CD105, a label that specifically binds to CD45, a label that specifically binds to CD34, a label that specifically binds to CD31, a label that specifically binds to VEGF2, a label that specifically binds to CD144, a label that specifically binds to CD106, or a combination thereof. In some embodiments, the systems further comprise anti-CD146 antibodies, anti-CD105 antibodies, anti-CD45 antibodies, anti CD34 antibodies, anti-CD31 antibodies, anti-VEGF2 antibodies, anti-CD 144 antibodies, anti-CD106 antibodies, or a combination thereof In some embodiments, the antibodies are labeled. In some embodiments, the antibodies are labeled with a fluorescent moiety or a magnetic moiety. In some embodiments, the systems further comprise a software module executed by the computer-processing device to transmit an analysis of the morphological feature of the population of CECs to the individual or a medical professional treating the individual. In some embodiments, the systems further comprise a software module executed by the computer-processing device to transmit a diagnosis or prognosis to the individual or a medical professional treating the individual. In some embodiments, the cardiovascular disorder is selected from: plaque rupture, plaque erosion, ischemia of the heart, reperfusion injury to the heart, atherosclerosis, or a combination thereof. In some embodiments, the cardiovascular disorder is acute coronary syndrome. In some embodiments, the cardiovascular disorder is selected from: ST-segment elevation myocardial infarction (STEMI), non-ST-segment elevation myocardial infarction (NSTEMI), unstable angina, or a combination thereof. In some embodiments, the cardiovascular disorder is ischemic stroke. In some embodiments, the cardiovascular disorder is atherosclerosis.

Disclosed herein, in certain embodiments, are methods of analyzing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard. In some embodiments, the method comprises comparing two morphological features of the population of circulating endothelial cells. In some embodiments, the method comprises comparing three morphological features of the population of circulating endothelial cells. In some embodiments, the method comprises comparing four morphological features of the population of circulating endothelial cells. In some embodiments, the morphological feature is: cellular area, nuclear area, ratio of cellular area to nuclear area, cell shape, nuclei shape, number of nuclei, number of circulating endothelial cells, or a combination thereof. In some embodiments, a software module executed by a computer-processing device compares the morphological feature. In some embodiments, the methods further comprise providing diagnostic or prognostic information to the individual about the cardiovascular disorder based on the comparison. In some embodiments, the methods further comprise diagnosing the individual with a cardiovascular disorder if the morphological feature(s) of the population of circulating endothelial cells (a) deviates from the control or standard from a healthy individual or population of healthy individuals, or (b) matches the control or standard from an individual or population of individuals who have or have had the cardiovascular disease. In some embodiments, the methods further comprise predicting the susceptibility of the individual for developing a cardiovascular disorder based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease. In some embodiments, the methods further comprise prescribing a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease. In some embodiments, the methods further comprise altering a treatment regimen prescribed or administered to the individual based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease. In some embodiments, the methods further comprise predicting the individual's response to a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease. In some embodiments, the deviation is the population of circulating endothelial cells having an average cellular area that is greater than the cellular area of CECs from a control or standard derived from a healthy individual or population of healthy individuals. In some embodiments, the deviation is the population of circulating endothelial cells having an average cellular area that is at least about 30% greater than the average cellular area of CECs from a control or standard derived from a healthy individual or population of healthy individuals. In some embodiments, the deviation is the population of circulating endothelial cells having an average cellular area that is greater than about 100 µm². In some embodiments, the deviation is the population of circulating endothelial cells having an average nuclear area that is greater than the nuclear area of CECs from a control or standard derived from a healthy individual or population of healthy individuals. In some embodiments, the deviation is the population of circulating endothelial cells having a average nuclear area that is at least about 30% greater than the average nuclear area of CECs from a control or standard derived from a healthy individual or population of healthy individuals. In some embodiments, the deviation is the population of circulating endothelial cells having an average nuclear area is greater than about 40 µm². In some embodiments, the deviation is the population of circulating endothelial cells having an average ratio of cellular area to nuclear area that is greater than the average ratio of cellular area to nuclear area of CECs from a control or standard derived from a healthy individual or population of healthy individuals. In some embodiments, the deviation is the population of circulating endothelial cells having an average number of nuclei that is greater than the number of nuclei of CECs from a control or standard derived from a healthy individual or population of healthy individuals. In some embodiments, the deviation is the population of circulating endothelial cells having an average of at least two nuclei per CEC. In some embodiments, the deviation is the population of circulating endothelial cells having an average of at least three nuclei per CEC. In some embodiments, the deviation is the population of circulating endothelial cells having an abnormal cellular shape as compared to the cellular shape of CECs from a control or standard derived from a healthy individual or population of healthy individual. In some embodiments, the deviation is the population of circulating endothelial cells having an abnormal nuclear shape as compared to the nuclear shape of CECs from a control or standard derived from a healthy individual or population of healthy individual. In some embodiments, the methods further comprise comparing: (a) the concentration of the population of circulating endothelial cells isolated from the blood sample; and (b) the concentration of a control or standard. In some embodiments, the methods further comprise using a machine to isolate the population of circulating endothelial cells from the blood sample. In some embodiments, the methods further comprise contacting the blood sample with a label that specifically binds to circulating endothelial cells, a label that does not bind to circulating endothelial cells, or a combination thereof. In some embodiments, the methods further comprise contacting the blood sample with a label that specifically binds to CD 146, a label that specifically binds to CD 105, a label that specifically binds to CD45, a label that specifically binds to CD34, a label that specifically binds to CD31, a label that specifically binds to VEGF2, a label that specifically binds to CD 144, a label that specifically binds to CD106, or a combination thereof. In some embodiments, the methods further comprise contacting the blood sample with anti-CD146 antibodies, anti-CD105 antibodies, anti-CD45 antibodies, anti CD34 antibodies, anti-CD31 antibodies, anti-VEGF2 antibodies, anti-CD144 antibodies, anti-CD106 antibodies or a combination thereof In some embodiments, the antibodies are labeled. In some embodiments, the antibodies are labeled with a fluorescent moiety or a magnetic moiety. In some embodiments, isolating the population of circulating endothelial cells from the blood sample comprises: (a) contacting the blood sample with anti-CD146 antibodies, wherein the antibodies are magnetically labeled or fluorescently labeled; (b) isolating the cells to which the anti-CD 146 antibodies specifically bind, to generate a population of CD146+ cells. In some embodiments, the methods further comprise contacting the population of CD 146+ cells with fluorescently labeled anti-CD45 antibodies, anti-CD105 antibodies, anti CD34 antibodies, anti-CD31 antibodies, anti-VEGF2 antibodies, anti-CD144 antibodies, anti-CD106 antibodies, DAPI, or a combination thereof. In some embodiments, the methods further comprise discarding a CD146+ cell that specifically binds to an anti-CD45 antibody and retaining a CD146+ cell that does not specifically bind to an anti-CD45 antibody, to generate a population of isolated circulating endothelial cells. In some embodiments, the cardiovascular disorder is selected from: plaque rupture, plaque erosion, ischemia of the heart, reperfusion injury to the heart, atherosclerosis, or a combination thereof. In some embodiments, the cardiovascular disorder is acute coronary syndrome. In some embodiments, the cardiovascular disorder is selected from: ST-segment elevation myocardial infarction (STEMI), non-ST-segment elevation myocardial infarction (NSTEMI), unstable angina, or a combination thereof. In some embodiments, the cardiovascular disorder is ischemic stroke. In some embodiments, the individual has been diagnosed with a cardiovascular disorder. In some embodiments, the individual is suspected of having a cardiovascular disorder. In some embodiments, the individual exhibits a symptom selected from: chest pain or discomfort; pain in the arms, neck, jaw, shoulder or back accompanying chest pain; nausea; fatigue; shortness of breath; sweating; dizziness; numbness or weakness of the face, arm or leg, especially on one side of the body; confusion, trouble speaking or understanding; trouble seeing in one or both eyes; trouble walking, dizziness, loss of balance or coordination; severe headache with no known cause or any combination thereof In some embodiments, the individual has no symptoms of a cardiovascular disorder.

Disclosed herein, in certain embodiments, are methods of diagnosing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard; and (c) diagnosis a cardiovascular disorder in the individual if the morphological feature(s) of the population of circulating endothelial cells (i) deviates from the control or standard from a healthy individual or population of healthy individuals, or (ii) matches the control or standard from an individual or population of individuals who have or have had the cardiovascular disease. In some embodiments, the methods comprise two morphological features of the population of circulating endothelial cells. In some embodiments, the methods comprise comparing three morphological features of the population of circulating endothelial cells. In some embodiments, the methods comprise comparing four morphological features of the population of circulating endothelial cells. In some embodiments, the morphological feature is: cellular area, nuclear area, ratio of cellular area to nuclear area, cell shape, nuclei shape, number of nuclei, number of circulating endothelial cells, or a combination thereof. In some embodiments, a software module executed by a computer-processing device compares the morphological feature of the population of circulating endothelial cells in the magnified image to the same morphological feature of the control or standard. In some embodiments, the methods further comprise prescribing a treatment regimen based on the diagnosis. In some embodiments, the methods further comprise altering a treatment regimen prescribed or administered to the individual based on the diagnosis. In some embodiments, the deviation is the population of circulating endothelial cells having an average cellular area that is greater than the cellular area of CECs from a control or standard derived from a healthy individual or population of healthy individuals. In some embodiments, the deviation is the population of circulating endothelial cells having an average cellular area that is at least about 30% greater than the average cellular area of CECs from a control or standard derived from a healthy individual or population of healthy individuals. In some embodiments, the deviation is the population of circulating endothelial cells having an average cellular area that is greater than about 100 µm². In some embodiments, the deviation is the population of circulating endothelial cells having an average nuclear area that is greater than the nuclear area of CECs from a control or standard derived from a healthy individual or population of healthy individuals. In some embodiments, the deviation is the population of circulating endothelial cells having a average nuclear area that is at least about 30% greater than the average nuclear area of CECs from a control or standard derived from a healthy individual or population of healthy individuals. In some embodiments, the deviation is the population of circulating endothelial cells having an average nuclear area is greater than about 40 µm². In some embodiments, the deviation is the population of circulating endothelial cells having an average ratio of cellular area to nuclear area that is greater than the average ratio of cellular area to nuclear area of CECs from a control or standard derived from a healthy individual or population of healthy individuals. In some embodiments, the deviation is the population of circulating endothelial cells having an average number of nuclei that is greater than the number of nuclei of CECs from a control or standard derived from a healthy individual or population of healthy individuals. In some embodiments, the deviation is the population of circulating endothelial cells having an average of at least two nuclei per CEC. In some embodiments, the deviation is the population of circulating endothelial cells having an average of at least three nuclei per CEC. In some embodiments, the deviation is the population of circulating endothelial cells having an abnormal cellular shape as compared to the cellular shape of CECs from a control or standard derived from a healthy individual or population of healthy individual. In some embodiments, the deviation is the population of circulating endothelial cells having an abnormal nuclear shape as compared to the nuclear shape of CECs from a control or standard derived from a healthy individual or population of healthy individual. In some embodiments, the methods further comprise comparing: (a) the concentration of the population of circulating endothelial cells isolated from the blood sample; and (b) the concentration of a control or standard. In some embodiments, the methods further comprise using a machine to isolate the population of circulating endothelial cells from the blood sample. In some embodiments, the methods further comprise contacting the blood sample with a label that specifically binds to circulating endothelial cells, a label that does not bind to circulating endothelial cells, or a combination thereof. In some embodiments, the methods further comprise contacting the blood sample with a label that specifically binds to CD 146, a label that specifically binds to CD 105, a label that specifically binds to CD45, a label that specifically binds to CD34, a label that specifically binds to CD31, a label that specifically binds to VEGF2, a label that specifically binds to CD 144, a label that specifically binds to CD 106, or a combination thereof. In some embodiments, the methods further comprise contacting the blood sample with anti-CD146 antibodies, anti-CD105 antibodies, anti-CD45 antibodies, anti CD34 antibodies, anti-CD31 antibodies, anti-VEGF2 antibodies, anti-CD144 antibodies, anti-CD106 antibodies or a combination thereof. In some embodiments, the antibodies are labeled. In some embodiments, the antibodies are labeled with a fluorescent moiety or a magnetic moiety. In some embodiments, isolating the population of circulating endothelial cells from the blood sample comprises: (a) contacting the blood sample with anti-CD146 antibodies, wherein the antibodies are magnetically labeled or fluorescently labeled; and (b) isolating the cells to which the anti-CD146 antibodies specifically bind, to generate a population of CD146+ cells. In some embodiments, the methods further comprise contacting the population of CD146+ cells with fluorescently labeled anti-CD45 antibodies, anti-CD105 antibodies, anti CD34 antibodies, anti-CD31 antibodies, anti-VEGF2 antibodies, anti-CD144 antibodies, anti-CD106 antibodies, DAPI, or a combination thereof In some embodiments, the methods further comprise discarding a CD 146+ cell that specifically binds to an anti-CD45 antibody and retaining a CD146+ cell that does not specifically bind to an anti-CD45 antibody, to generate a population of isolated circulating endothelial cells. In some embodiments, the cardiovascular disorder is selected from: plaque rupture, plaque erosion, ischemia of the heart, reperfusion injury to the heart, atherosclerosis, or a combination thereof In some embodiments, the cardiovascular disorder is acute coronary syndrome. In some embodiments, the cardiovascular disorder is selected from: ST-segment elevation myocardial infarction (STEMI), non-ST-segment elevation myocardial infarction (NSTEMI), unstable angina, or a combination thereof In some embodiments, the cardiovascular disorder is ischemic stroke. In some embodiments, the individual has been diagnosed with a cardiovascular disorder. In some embodiments, the individual is suspected of having a cardiovascular disorder. In some embodiments, the individual exhibits a symptom selected from: chest pain or discomfort; pain in the arms, neck, jaw, shoulder or back accompanying chest pain; nausea; fatigue; shortness of breath; sweating; dizziness; numbness or weakness of the face, arm or leg, especially on one side of the body; confusion, trouble speaking or understanding; trouble seeing in one or both eyes; trouble walking, dizziness, loss of balance or coordination; severe headache with no known cause or any combination thereof. In some embodiments, the individual has no symptoms of a cardiovascular disorder.

Disclosed herein, in certain embodiments, are methods of predicting whether an individual is susceptible to developing a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the susceptibility of the individual for developing a cardiovascular disorder based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. In some embodiments, the methods comprise two morphological features of the population of circulating endothelial cells. In some embodiments, the methods comprise comparing three morphological features of the population of circulating endothelial cells. In some embodiments, the methods comprise comparing four morphological features of the population of circulating endothelial cells. In some embodiments, the morphological feature is: cellular area, nuclear area, ratio of cellular area to nuclear area, cell shape, nuclei shape, number of nuclei, number of circulating endothelial cells, or a combination thereof. In some embodiments, a software module executed by a computer-processing device compares the morphological feature of the population of circulating endothelial cells in the magnified image to the same morphological feature of the control or standard. In some embodiments, the methods further comprise prescribing a treatment regimen based on the diagnosis. In some embodiments, the methods further comprise altering a treatment regimen prescribed or administered to the individual based on the diagnosis. In some embodiments, the deviation is the population of circulating endothelial cells having an average cellular area that is greater than the cellular area of CECs from a control or standard derived from a healthy individual or population of healthy individuals. In some embodiments, the deviation is the population of circulating endothelial cells having an average cellular area that is at least about 30% greater than the average cellular area of CECs from a control or standard derived from a healthy individual or population of healthy individuals. In some embodiments, the deviation is the population of circulating endothelial cells having an average cellular area that is greater than about 100 µm². In some embodiments, the deviation is the population of circulating endothelial cells having an average nuclear area that is greater than the nuclear area of CECs from a control or standard derived from a healthy individual or population of healthy individuals. In some embodiments, the deviation is the population of circulating endothelial cells having a average nuclear area that is at least about 30% greater than the average nuclear area of CECs from a control or standard derived from a healthy individual or population of healthy individuals. In some embodiments, the deviation is the population of circulating endothelial cells having an average nuclear area is greater than about 40 µm². In some embodiments, the deviation is the population of circulating endothelial cells having an average ratio of cellular area to nuclear area that is greater than the average ratio of cellular area to nuclear area of CECs from a control or standard derived from a healthy individual or population of healthy individuals. In some embodiments, the deviation is the population of circulating endothelial cells having an average number of nuclei that is greater than the number of nuclei of CECs from a control or standard derived from a healthy individual or population of healthy individuals. In some embodiments, the deviation is the population of circulating endothelial cells having an average of at least two nuclei per CEC. In some embodiments, the deviation is the population of circulating endothelial cells having an average of at least three nuclei per CEC. In some embodiments, the deviation is the population of circulating endothelial cells having an abnormal cellular shape as compared to the cellular shape of CECs from a control or standard derived from a healthy individual or population of healthy individual. In some embodiments, the deviation is the population of circulating endothelial cells having an abnormal nuclear shape as compared to the nuclear shape of CECs from a control or standard derived from a healthy individual or population of healthy individual. In some embodiments, the methods further comprise comparing: (a) the concentration of the population of circulating endothelial cells isolated from the blood sample; and (b) the concentration of a control or standard. In some embodiments, the methods further comprise using a machine to isolate the population of circulating endothelial cells from the blood sample. In some embodiments, the methods further comprise contacting the blood sample with a label that specifically binds to circulating endothelial cells, a label that does not bind to circulating endothelial cells, or a combination thereof. In some embodiments, the methods further comprise contacting the blood sample with a label that specifically binds to CD146, a label that specifically binds to CD105, a label that specifically binds to CD45, a label that specifically binds to CD34, a label that specifically binds to CD31, a label that specifically binds to VEGF2, a label that specifically binds to CD 144, a label that specifically binds to CD106, or a combination thereof. In some embodiments, the methods further comprise contacting the blood sample with anti-CD146 antibodies, anti-CD105 antibodies, anti-CD45 antibodies, anti CD34 antibodies, anti-CD31 antibodies, anti-VEGF2 antibodies, anti-CD144 antibodies, anti-CD106 antibodies or a combination thereof In some embodiments, the antibodies are labeled. In some embodiments, the antibodies are labeled with a fluorescent moiety or a magnetic moiety. In some embodiments, isolating the population of circulating endothelial cells from the blood sample comprises: (a) contacting the blood sample with anti-CD 146 antibodies, wherein the antibodies are magnetically labeled or fluorescently labeled; and (b) isolating the cells to which the anti-CD146 antibodies specifically bind, to generate a population of CD146+ cells. In some embodiments, the methods further comprise contacting the population of CD146+ cells with fluorescently labeled anti-CD45 antibodies, anti-CD105 antibodies, anti CD34 antibodies, anti-CD31 antibodies, anti-VEGF2 antibodies, anti-CD144 antibodies, anti-CD106 antibodies, DAPI, or a combination thereof. In some embodiments, the methods further comprise discarding a CD146+ cell that specifically binds to an anti-CD45 antibody and retaining a CD146+ cell that does not specifically bind to an anti-CD45 antibody, to generate a population of isolated circulating endothelial cells. In some embodiments, the cardiovascular disorder is selected from: plaque rupture, plaque erosion, ischemia of the heart, reperfusion injury to the heart, atherosclerosis, or a combination thereof. In some embodiments, the cardiovascular disorder is acute coronary syndrome. In some embodiments, the cardiovascular disorder is selected from: ST-segment elevation myocardial infarction (STEMI), non-ST-segment elevation myocardial infarction (NSTEMI), unstable angina, or a combination thereof. In some embodiments, the cardiovascular disorder is ischemic stroke. In some embodiments, the individual has been diagnosed with a cardiovascular disorder. In some embodiments, the individual is suspected of having a cardiovascular disorder. In some embodiments, the individual exhibits a symptom selected from: chest pain or discomfort; pain in the arms, neck, jaw, shoulder or back accompanying chest pain; nausea; fatigue; shortness of breath; sweating; dizziness; numbness or weakness of the face, arm or leg, especially on one side of the body; confusion, trouble speaking or understanding; trouble seeing in one or both eyes; trouble walking, dizziness, loss of balance or coordination; severe headache with no known cause or any combination thereof. In some embodiments, the individual has no symptoms of a cardiovascular disorder.

Disclosed herein, in certain embodiments, are methods of predicting an individual's response to a treatment regimen for a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the individual's response to a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease. In some embodiments, the methods comprise two morphological features of the population of circulating endothelial cells. In some embodiments, the methods comprise comparing three morphological features of the population of circulating endothelial cells. In some embodiments, the methods comprise comparing four morphological features of the population of circulating endothelial cells. In some embodiments, the morphological feature is: cellular area, nuclear area, ratio of cellular area to nuclear area, cell shape, nuclei shape, number of nuclei, number of circulating endothelial cells, or a combination thereof In some embodiments, a software module executed by a computer-processing device compares the morphological feature of the population of circulating endothelial cells in the magnified image to the same morphological feature of the control or standard. In some embodiments, the methods further comprise prescribing a treatment regimen based on the diagnosis. In some embodiments, the methods further comprise altering a treatment regimen prescribed or administered to the individual based on the diagnosis. In some embodiments, the deviation is the population of circulating endothelial cells having an average cellular area that is greater than the cellular area of CECs from a control or standard derived from a healthy individual or population of healthy individuals. In some embodiments, the deviation is the population of circulating endothelial cells having an average cellular area that is at least about 30% greater than the average cellular area of CECs from a control or standard derived from a healthy individual or population of healthy individuals. In some embodiments, the deviation is the population of circulating endothelial cells having an average cellular area that is greater than about 100 µm². In some embodiments, the deviation is the population of circulating endothelial cells having an average nuclear area that is greater than the nuclear area of CECs from a control or standard derived from a healthy individual or population of healthy individuals. In some embodiments, the deviation is the population of circulating endothelial cells having a average nuclear area that is at least about 30% greater than the average nuclear area of CECs from a control or standard derived from a healthy individual or population of healthy individuals. In some embodiments, the deviation is the population of circulating endothelial cells having an average nuclear area is greater than about 40 µm². In some embodiments, the deviation is the population of circulating endothelial cells having an average ratio of cellular area to nuclear area that is greater than the average ratio of cellular area to nuclear area of CECs from a control or standard derived from a healthy individual or population of healthy individuals. In some embodiments, the deviation is the population of circulating endothelial cells having an average number of nuclei that is greater than the number of nuclei of CECs from a control or standard derived from a healthy individual or population of healthy individuals. In some embodiments, the deviation is the population of circulating endothelial cells having an average of at least two nuclei per CEC. In some embodiments, the deviation is the population of circulating endothelial cells having an average of at least three nuclei per CEC. In some embodiments, the deviation is the population of circulating endothelial cells having an abnormal cellular shape as compared to the cellular shape of CECs from a control or standard derived from a healthy individual or population of healthy individual. In some embodiments, the deviation is the population of circulating endothelial cells having an abnormal nuclear shape as compared to the nuclear shape of CECs from a control or standard derived from a healthy individual or population of healthy individual. In some embodiments, the methods further comprise comparing: (a) the concentration of the population of circulating endothelial cells isolated from the blood sample; and (b) the concentration of a control or standard. In some embodiments, the methods further comprise using a machine to isolate the population of circulating endothelial cells from the blood sample. In some embodiments, the methods further comprise contacting the blood sample with a label that specifically binds to circulating endothelial cells, a label that does not bind to circulating endothelial cells, or a combination thereof In some embodiments, the methods further comprise contacting the blood sample with a label that specifically binds to CD146, a label that specifically binds to CD105, a label that specifically binds to CD45, a label that specifically binds to CD34, a label that specifically binds to CD31, a label that specifically binds to VEGF2, a label that specifically binds to CD144, a label that specifically binds to CD106, or a combination thereof. In some embodiments, the methods further comprise contacting the blood sample with anti-CD 146 antibodies, anti-CD105 antibodies, anti-CD45 antibodies, anti CD34 antibodies, anti-CD31 antibodies, anti-VEGF2 antibodies, anti-CD144 antibodies, anti-CD106 antibodies or a combination thereof. In some embodiments, the antibodies are labeled. In some embodiments, the antibodies are labeled with a fluorescent moiety or a magnetic moiety. In some embodiments, isolating the population of circulating endothelial cells from the blood sample comprises: (a) contacting the blood sample with anti-CD146 antibodies, wherein the antibodies are magnetically labeled or fluorescently labeled; and (b) isolating the cells to which the anti-CD146 antibodies specifically bind, to generate a population of CD 146+ cells. In some embodiments, the methods further comprise contacting the population of CD146+ cells with fluorescently labeled anti-CD45 antibodies, anti-CD105 antibodies, anti CD34 antibodies, anti-CD31 antibodies, anti-VEGF2 antibodies, anti-CD144 antibodies, anti-CD106 antibodies, DAPI, or a combination thereof In some embodiments, the methods further comprise discarding a CD146+ cell that specifically binds to an anti-CD45 antibody and retaining a CD 146+ cell that does not specifically bind to an anti-CD45 antibody, to generate a population of isolated circulating endothelial cells. In some embodiments, the cardiovascular disorder is selected from: plaque rupture, plaque erosion, ischemia of the heart, reperfusion injury to the heart, atherosclerosis, or a combination thereof. In some embodiments, the cardiovascular disorder is acute coronary syndrome. In some embodiments, the cardiovascular disorder is selected from: ST-segment elevation myocardial infarction (STEMI), non-ST-segment elevation myocardial infarction (NSTEMI), unstable angina, or a combination thereof. In some embodiments, the cardiovascular disorder is ischemic stroke. In some embodiments, the individual has been diagnosed with a cardiovascular disorder. In some embodiments, the individual is suspected of having a cardiovascular disorder. In some embodiments, the individual exhibits a symptom selected from: chest pain or discomfort; pain in the arms, neck, jaw, shoulder or back accompanying chest pain; nausea; fatigue; shortness of breath; sweating; dizziness; numbness or weakness of the face, arm or leg, especially on one side of the body; confusion, trouble speaking or understanding; trouble seeing in one or both eyes; trouble walking, dizziness, loss of balance or coordination; severe headache with no known cause or any combination thereof. In some embodiments, the individual has no symptoms of a cardiovascular disorder.

Disclosed herein, in certain embodiments, are methods of prescribing a treatment regimen for a cardiovascular disorder to an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) prescribing a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease. In some embodiments, the methods comprise two morphological features of the population of circulating endothelial cells. In some embodiments, the methods comprise comparing three morphological features of the population of circulating endothelial cells. In some embodiments, the methods comprise comparing four morphological features of the population of circulating endothelial cells. In some embodiments, the morphological feature is: cellular area, nuclear area, ratio of cellular area to nuclear area, cell shape, nuclei shape, number of nuclei, number of circulating endothelial cells, or a combination thereof. In some embodiments, a software module executed by a computer-processing device compares the morphological feature of the population of circulating endothelial cells in the magnified image to the same morphological feature of the control or standard. In some embodiments, the methods further comprise prescribing a treatment regimen based on the diagnosis. In some embodiments, the methods further comprise altering a treatment regimen prescribed or administered to the individual based on the diagnosis. In some embodiments, the deviation is the population of circulating endothelial cells having an average cellular area that is greater than the cellular area of CECs from a control or standard derived from a healthy individual or population of healthy individuals. In some embodiments, the deviation is the population of circulating endothelial cells having an average cellular area that is at least about 30% greater than the average cellular area of CECs from a control or standard derived from a healthy individual or population of healthy individuals. In some embodiments, the deviation is the population of circulating endothelial cells having an average cellular area that is greater than about 100 µm². In some embodiments, the deviation is the population of circulating endothelial cells having an average nuclear area that is greater than the nuclear area of CECs from a control or standard derived from a healthy individual or population of healthy individuals. In some embodiments, the deviation is the population of circulating endothelial cells having a average nuclear area that is at least about 30% greater than the average nuclear area of CECs from a control or standard derived from a healthy individual or population of healthy individuals. In some embodiments, the deviation is the population of circulating endothelial cells having an average nuclear area is greater than about 40 µm². In some embodiments, the deviation is the population of circulating endothelial cells having an average ratio of cellular area to nuclear area that is greater than the average ratio of cellular area to nuclear area of CECs from a control or standard derived from a healthy individual or population of healthy individuals. In some embodiments, the deviation is the population of circulating endothelial cells having an average number of nuclei that is greater than the number of nuclei of CECs from a control or standard derived from a healthy individual or population of healthy individuals. In some embodiments, the deviation is the population of circulating endothelial cells having an average of at least two nuclei per CEC. In some embodiments, the deviation is the population of circulating endothelial cells having an average of at least three nuclei per CEC. In some embodiments, the deviation is the population of circulating endothelial cells having an abnormal cellular shape as compared to the cellular shape of CECs from a control or standard derived from a healthy individual or population of healthy individual. In some embodiments, the deviation is the population of circulating endothelial cells having an abnormal nuclear shape as compared to the nuclear shape of CECs from a control or standard derived from a healthy individual or population of healthy individual. In some embodiments, the methods further comprise comparing: (a) the concentration of the population of circulating endothelial cells isolated from the blood sample; and (b) the concentration of a control or standard. In some embodiments, the methods further comprise using a machine to isolate the population of circulating endothelial cells from the blood sample. In some embodiments, the methods further comprise contacting the blood sample with a label that specifically binds to circulating endothelial cells, a label that does not bind to circulating endothelial cells, or a combination thereof. In some embodiments, the methods further comprise contacting the blood sample with a label that specifically binds to CD146, a label that specifically binds to CD105, a label that specifically binds to CD45, a label that specifically binds to CD34, a label that specifically binds to CD31, a label that specifically binds to VEGF2, a label that specifically binds to CD144, a label that specifically binds to CD106, or a combination thereof. In some embodiments, the methods further comprise contacting the blood sample with anti-CD146 antibodies, anti-CD105 antibodies, anti-CD45 antibodies, anti CD34 antibodies, anti-CD31 antibodies, anti-VEGF2 antibodies, anti-CD 144 antibodies, anti-CD106 antibodies or a combination thereof. In some embodiments, the antibodies are labeled. In some embodiments, the antibodies are labeled with a fluorescent moiety or a magnetic moiety. In some embodiments, isolating the population of circulating endothelial cells from the blood sample comprises: (a) contacting the blood sample with anti-CD 146 antibodies, wherein the antibodies are magnetically labeled or fluorescently labeled; and (b) isolating the cells to which the anti-CD146 antibodies specifically bind, to generate a population of CD146+ cells. In some embodiments, the methods further comprise contacting the population of CD146+ cells with fluorescently labeled anti-CD45 antibodies, anti-CD105 antibodies, anti CD34 antibodies, anti-CD31 antibodies, anti-VEGF2 antibodies, anti-CD144 antibodies, anti-CD106 antibodies, DAPI, or a combination thereof. In some embodiments, the methods further comprise discarding a CD146+ cell that specifically binds to an anti-CD45 antibody and retaining a CD 146+ cell that does not specifically bind to an anti-CD45 antibody, to generate a population of isolated circulating endothelial cells. In some embodiments, the cardiovascular disorder is selected from: plaque rupture, plaque erosion, ischemia of the heart, reperfusion injury to the heart, atherosclerosis, or a combination thereof. In some embodiments, the cardiovascular disorder is acute coronary syndrome. In some embodiments, the cardiovascular disorder is selected from: ST-segment elevation myocardial infarction (STEMI), non-ST-segment elevation myocardial infarction (NSTEMI), unstable angina, or a combination thereof. In some embodiments, the cardiovascular disorder is ischemic stroke. In some embodiments, the individual has been diagnosed with a cardiovascular disorder. In some embodiments, the individual is suspected of having a cardiovascular disorder. In some embodiments, the individual exhibits a symptom selected from: chest pain or discomfort; pain in the arms, neck, jaw, shoulder or back accompanying chest pain; nausea; fatigue; shortness of breath; sweating; dizziness; numbness or weakness of the face, arm or leg, especially on one side of the body; confusion, trouble speaking or understanding; trouble seeing in one or both eyes; trouble walking, dizziness, loss of balance or coordination; severe headache with no known cause or any combination thereof. In some embodiments, the individual has no symptoms of a cardiovascular disorder.

Disclosed herein, in certain embodiments, is an isolated circulating endothelial cell (CEC) comprising at least one morphological feature that (a) deviates from the control or standard from a healthy individual or population of healthy individuals, or (b) matches the control or standard from an individual or population of individuals who have or have had a cardiovascular disease of interest. In some embodiments, the CEC comprises two morphological features that (a) deviates from the control or standard from a healthy individual or population of healthy individuals, or (b) matches the control or standard from an individual or population of individuals who have or have had a cardiovascular disease of interest: In some embodiments, the CEC comprises three morphological features that (a) deviates from the control or standard from a healthy individual or population of healthy individuals, or (b) matches the control or standard from an individual or population of individuals who have or have had a cardiovascular disease of interest. In some embodiments, the CEC comprises four morphological features that (a) deviates from the control or standard from a healthy individual or population of healthy individuals, or (b) matches the control or standard from an individual or population of individuals who have or have had a cardiovascular disease of interest. In some embodiments, the deviation is a qualitative deviation, a quantitative deviation or a combination thereof. In some embodiments, the morphological feature(s) is selected from: cellular area, nuclear area, ratio of cellular area to nuclear area, cell shape, nuclei shape, and number of nuclei. In some embodiments, the isolated circulating endothelial cell is multinucleated. In some embodiments, the isolated circulating endothelial cell comprises three nuclei. In some embodiments, the nuclear area of the isolated circulating endothelial cell is at least about 30% larger than the nuclear area of a standard or control. In some embodiments, the cellular area of the isolated circulating endothelial cell is at least about 30% larger than the cellular area of a standard or control. In some embodiments, the isolated circulating endothelial cell is isolated from an individual diagnosed with a cardiovascular disorder. In some embodiments, the isolated circulating endothelial cell is isolated from an individual suspected of having a cardiovascular disorder. In some embodiments, the isolated circulating endothelial cell is isolated from an individual with no symptoms of a cardiovascular disorder. In some embodiments, the circulating endothelial cell is isolated by use of a machine. In some embodiments, the circulating endothelial cell is isolated by: (a) contacting the blood sample with anti-CD 146 antibodies, wherein the antibodies are magnetically labeled or fluorescently labeled; and (b) isolating the cells to which the anti-CD146 antibodies specifically bind, to generate a population of CD146+ cells. In some embodiments, the circulating endothelial cell is isolated by: (a) contacting the blood sample with anti-CD146 antibodies, wherein the antibodies are magnetically labeled or fluorescently labeled; (b) isolating the cells to which the anti-CD146 antibodies specifically bind, to generate a population of CD146+ cells; and (c) contacting the population of CD146+ cells with fluorescently labeled anti-CD45 antibodies, anti-CD105 antibodies, anti CD34 antibodies, anti-CD31 antibodies, anti-VEGF2 antibodies, anti-CD144 antibodies, anti-CD106 antibodies, DAPI, or a combination thereof

### FIGURES

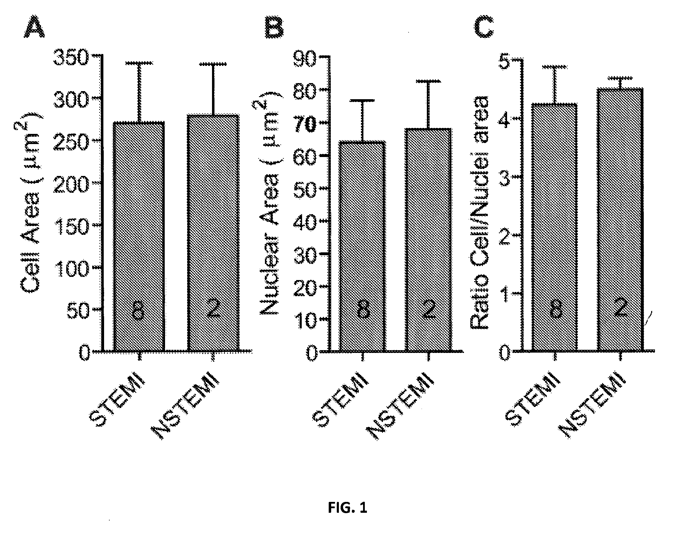

Fig. 1, provides illustrative CEC size analysis for STEMI and NSTEMI patients. (A) Cellular areas (µm²) for cells from each patient group (mean ± SD). P-value: 0.8842. (B) Nuclear areas (µm²) for cells from each patient group (mean ± SD). P-value: 0.7114. (C) Ratios between the cellular area and the nuclear area for cells from each patient group (mean ± SD). P-value: 0.6131. Numbers in each bar for A-C represent the number of patients analyzed from each group; all CECs from each control individual and 50-100 CECs randomly selected from each MI patient were analyzed.

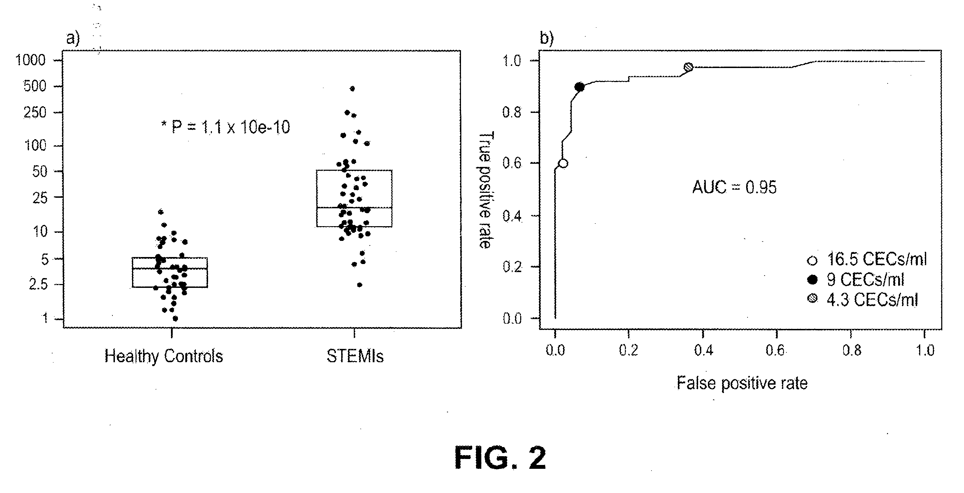

Fig. 2, provides an illustrative enumeration of CECs in healthy controls and STEMI patients. (A) CEC quantification in 44 consecutive healthy control and 50 STEMI patients. The median number of CECs in healthy controls was 4 CECs/ml with upper and lower quartiles of 2 and 5 CECs/ml, respectively. The median number of CECs in STEMI patients was 19 with upper and lower quartiles of 12 and 51 CECs/ml (p = 1.1x10-10), respectively. (B) Area under the receiver operating characteristic curve (AUC) was equal to 0.95. The red point represents a classification threshold of 16.5 CECs/ml, which is associated with a sensitivity of 60% and specificity of 98%; blue equals a classification threshold of 9 CECs/ml, with an associated sensitivity of 90% and specificity of 93%; and green equals to a threshold of 4.3 CECs/ml with a 98% sensitivity and 64% specificity for correctly classifying an MI case or control.

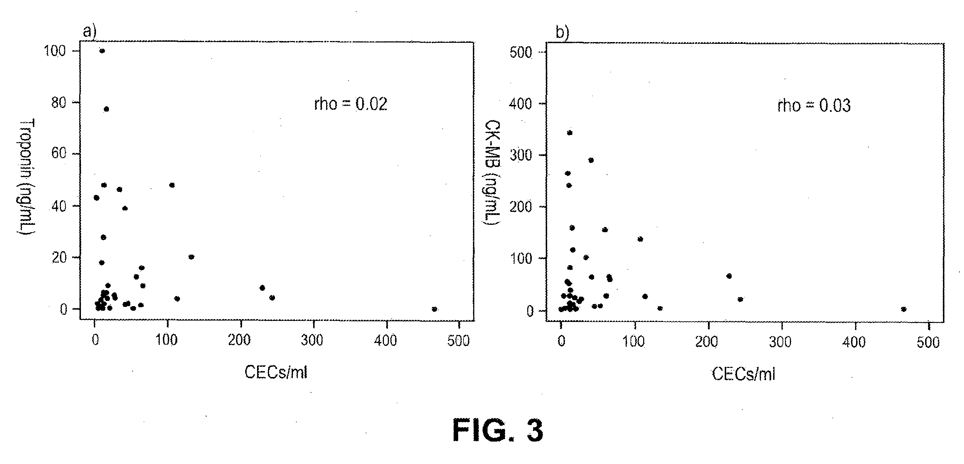

Fig. 3, provides an illustrative correlation between CEC counts and typical markers of myocardial necrosis. (A) Using spearman's rank correlation (rho) coefficient, CEC counts in MI patients were assessed for correlation to initial presenting serum cardiac troponin values. No evidence of correlation was seen (rho = 0.02, p = 0.9). (B) No correlation between CEC counts and initial CK-MB values were noted (rho = 0.03, p = 0.9).

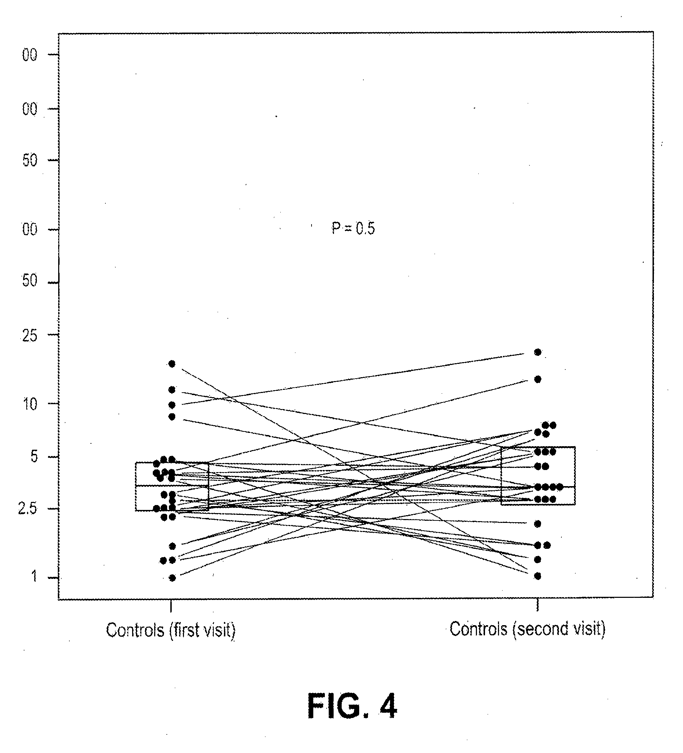

Fig. 4, provides an example of the stability of CEC counts over time. Two repeat CEC measurements in 25 consecutive control individuals at visits separated by a 2-month time frame. Measurements performed on the same individual are connected with a gray line. The CEC counts measured at first visit ranged from 1 to 16.75 CECs/ml, with a median value of 3.38 CECs/ml. On the second visit, the CEC counts measured in the same 24 individuals ranged from 1 to 19.5 CECs/ml, with a median value equal to 3.25 CECs/ml. Using paired Wilcoxon signed rank test, we found no evidence of a difference between the CEC counts at the two time points.

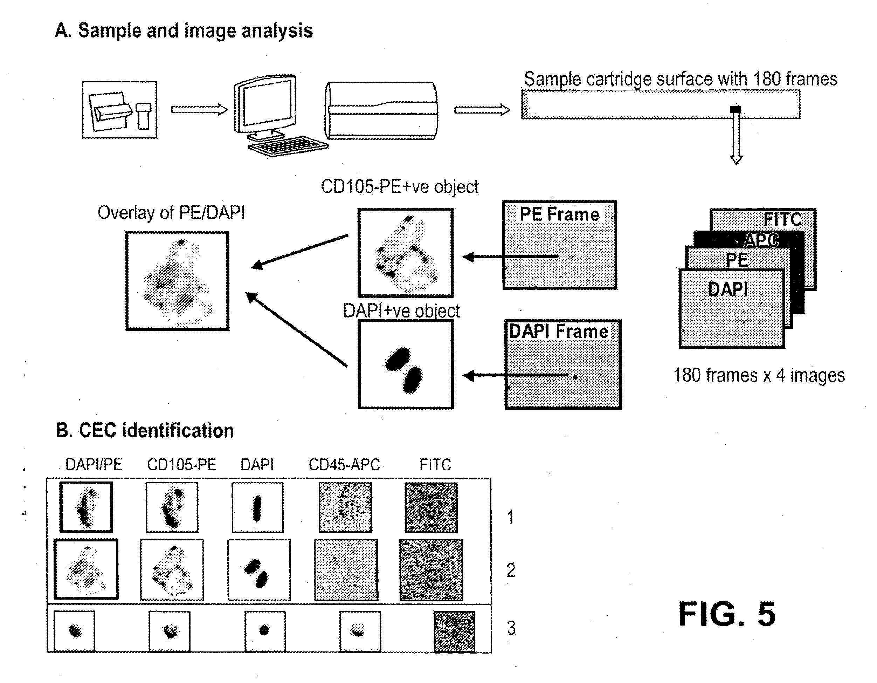

Fig. 5, provides an illustrative image analysis by the CellTracks Analyzer II® and identification of CECs (A) The steps required for sample and image analysis by the CTA II. The Magnest containing the sample processed in the AutoPrep is placed into the CTA II. The CTA II records images in each of 4 colors for the entire working surface of the sample chamber. The software module then selects objects within these images that are larger than 4µm, and appear to be positive for both DAPI and CD105-PE. These selected candidate CEC images are presented as a series of thumbnail Browser images for a trained reviewer to identify and score as CECs, or dismiss as non-CEC cells or related materials as shown in B. (B) Browser images of objects detected and presented as possible candidate CECs by the CTA II. In this example, 3 objects are presented to the reviewer. The thumbnail images show, from right to left, an unused (FITC) channel, CD45-APC signal, DAPI stained nuclei, CD105-PE reactivity, and finally an overlay of the CD105-PE & DAPI staining. To be scored as a CEC, a cell must have a nucleus, express CD105, have the morphology of a cell, and be negative for CD45. The first two objects met these criteria and were scored as CECs (checked box). The third object was judged a leukocyte because it was CD45 positive (box not checked). The software module automatically tabulates the checked boxes within each sample, and results are expressed as the number of CECs per 4 mL of blood.

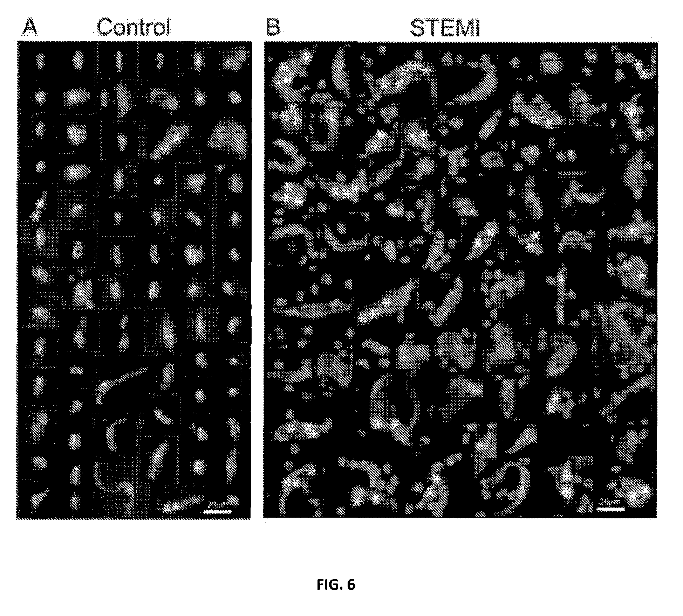

Fig. 6, provides illustrative fluorescence microscopy images of individual CECs isolated from (A) age-matched controls, and (B) a representative STEMI patient (CEC03040). CECs are labeled with CD105 (green) and DAPI (purple) as markers for their cellular and nuclear compartments, respectively. CECs are identified in these images based on co-localization of CD105 and DAPI staining, resulting in a green cell body overlaying a purple nucleus. Purple nuclei not co-localized with green CD105 staining are contaminating white blood cells also present in the field of view (based on positive staining for CD45, not shown). CECs from the STEMI patient are heterogeneous in size and shape and many of them are much larger than CECs from controls, with multiple nuclei. Image magnification is constant for all images. All CEC images from 3 age-matched control individuals are shown, and 69 CEC images were randomly selected for presentation from STEMI patient CEC03040, who had 58 CECs/ml of blood, in the mid range of CECs/ml for the MI patients in this study. Bars, 20µm. Asterisks, Multiple nuclei associated with one CD105-stained cell body.

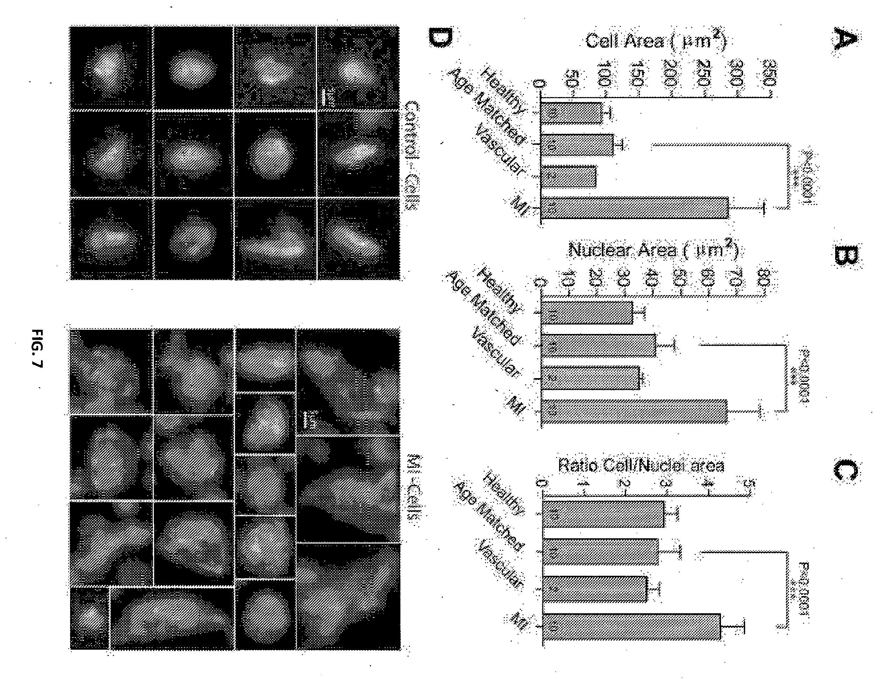

Fig. 7, provides an illustrative CEC size analysis for control (age-matched and healthy), vascular and MI (STEMI and NSTEMI) patients. (A) Cellular areas (µm²) for cells from each group (mean ± SD). P-values: 0.0133 (healthy vs. age-matched); <0.0001 (age-matched vs. MI); 0.003 (vascular vs. MI). (B) Nuclear areas (µm²) for cells from each group (mean ± SD). P-values: 0.0069 (healthy vs. age- matched); <0.0001 (aged matched vs. MI); 0.0059 (vascular vs. MI). (C) Ratios between the cellular area and the nuclear area for cells from each patient group (mean ± SD). P-values: 0.4848 (healthy vs. age-matched); <0.0001 (age-matched vs. MI); 0.0021 (vascular vs. MI). Numbers in each bar for A-C represent the number of patients analyzed from each group; all CECs from each control individual and 50-100 CECs randomly selected from each MI patient were analyzed. Image magnification is constant for all images. Bars, 5µm. (D) Representative images of CECs from control (age-matched and healthy) and MI (STEMI and NSTEMI) patients, illustrating typical CECs (i.e., mean area sizes), and the range of variation in CEC area sizes from these groups. CECs were identified with CD105 (green) and DAPI (purple) staining as markers for cellular and nuclear compartments respectively.

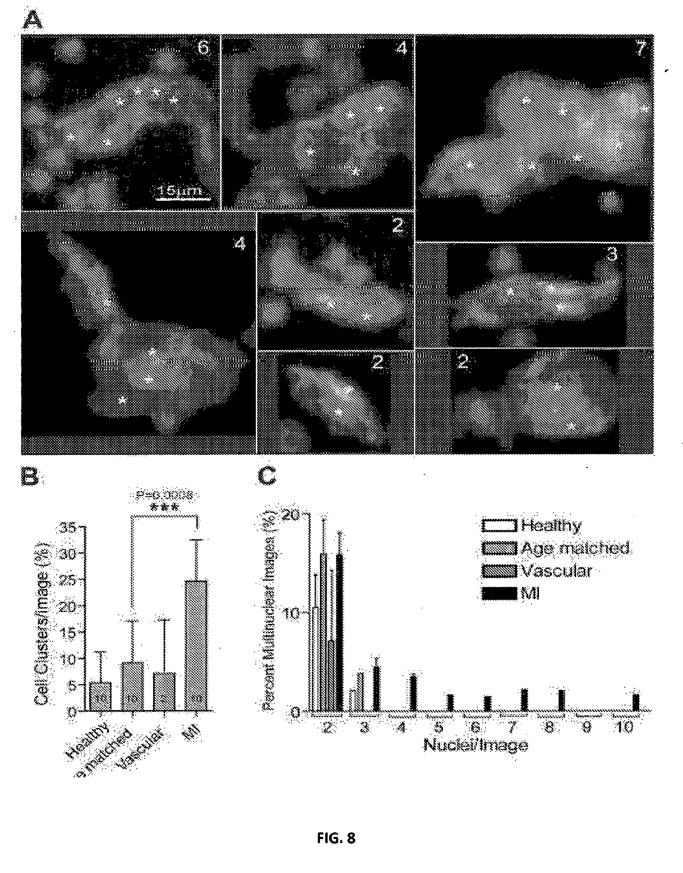

Fig. 8, provides an illustrative analysis of numbers of CECs with multiple nuclei from control, random age-matched population sample, vascular, and MI (STEMI and NSTEMI) patients. (A) Images of CECs with multiple nuclei from the STEMI patient group stained with CD105 (green) and DAPI (purple, white asterisks) to identify the cellular and the nuclear compartments, respectively. The number of nuclei per CD105-stained cell body is specified in the upper right of each image. Image magnification is constant for all images. Bar, 15µm. (B) Percent of CEC images with multiple nuclei (two or more nuclei/image) in each group (mean ± SD). The numbers in each bar represent the number of individuals analyzed. (C) Distribution of number of nuclei/image for the different groups (mean ± SD). Bars indicate Healthy (white) and Age-matched (grey) controls, Vascular (red) and MI (black) patients. P-values: 0.2295 (healthy vs age-matched); 0.0008 (age-matched vs MI); 0.0231 (vascular vs MI).

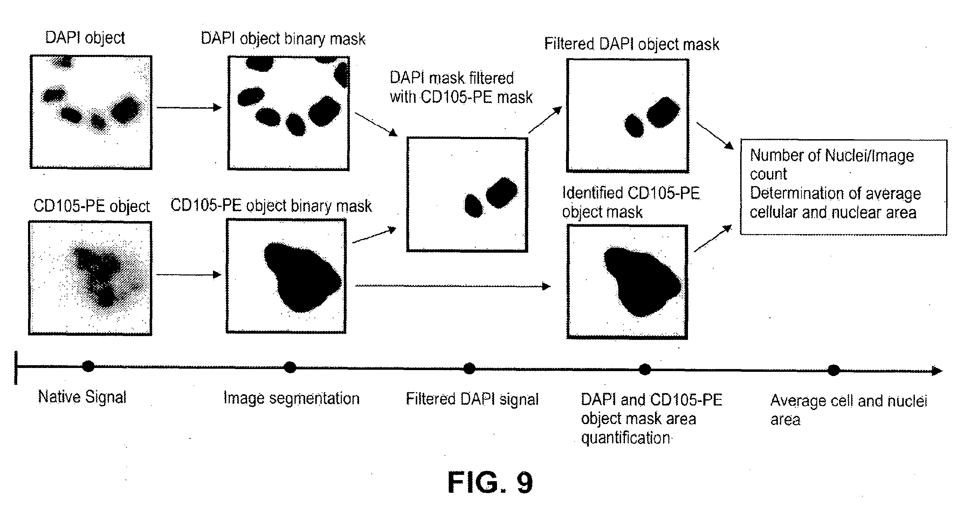

Fig. 9, provides an example of automated image processing for detection and quantification of CEC cell body and nuclei captured by Celltracks analyzer II. CECs isolated by Celltracks analyzer II from patients and controls were fixed and stained for DNA content (DAPI) and CD105 expression (CD-105-PE). The schematic diagram shows the steps involved in the automated imaging process used for determination of numbers of nuclei/image and cellular and nuclear areas. After acquisition the native signal was processed with custom software module to generate a segmented image (see Methods). Nuclei not associated with CECs were eliminated by comparison of the binary masks of the DAPI and CD105 channels. Areas for the segmented image channels were calculated from the filtered nuclear and cellular binary masks. The number of detected objects in the segmented DAPI channel (associated with CECs) was determined and used to average the nuclear and cellular area for every image.

### DETAILED DESCRIPTION OF THE INVENTION

Serious cardiovascular events (such as myocardial infarction (MI) and ischemic stroke) are often fatal. Alternatively, where there are survivors, these events may leave the survivors with lasting, and quality of life decreasing effects (e.g., partial or complete paralysis, aphasia, memory loss). Many serious cardiovascular events are induced by arterial plaque rupture. In many cases, there are no signs of an impending rupture or the signs are inconclusive. For example, cardiac troponins that mark myocardial necrosis but not arterial injury are lacking in specificity and may show an elevated signal in a number of conditions unrelated to MI, including atrial fibrillation, pneumonia, sepsis, pulmonary embolism, and chronic kidney disease. Further, in certain types of myocardial infarction (NSTEMI), there are no changes on an electrocardiogram (ECG).

Given the significant dangers associated with serious cardiovascular events and their unpredictable nature, there is a need for methods of identifying individuals who are at risk for arterial plaque rupture before they are clinically manifested. Further, these methods should be non-invasive, easily implemented (e.g., in clinical settings), and easy to interpret.

Disclosed herein, in certain embodiments, are methods of predicting arterial plaque rupture before it is clinically manifested comprising analyzing CECs.

Disclosed herein, in certain embodiments, are methods of analyzing a cardiovascular disorder in an individual in need thereof, comprising: comparing a morphological feature of: (a) a population of circulating endothelial cells isolated from a blood sample obtained from the individual, wherein the blood sample comprises cells selected from circulating endothelial cells and circulating non-endothelial cells; and (b) control or standard.

Further disclosed herein, in certain embodiments, are methods of diagnosing a cardiovascular disorder in an individual in need thereof, comprising: (a) comparing a morphological feature of: (i) a population of circulating endothelial cells isolated from a blood sample obtained from the individual, wherein the blood sample comprises cells selected from circulating endothelial cells and circulating non-endothelial cells; and (ii) control or standard; and (b) diagnosing the individual with a cardiovascular disorder if the morphological feature of the population of circulating endothelial cells deviates from the control or standard by at least a predetermined amount.

Additionally, disclosed herein, in certain embodiments, are methods of predicting that an individual is susceptible to developing a cardiovascular disorder, comprising: (a) comparing a morphological feature of: (i) a population of circulating endothelial cells isolated from a blood sample obtained from the individual, wherein the blood sample comprises cells selected from circulating endothelial cells and circulating non-endothelial cells; and (ii) control or standard; and (b) predicting that the individual is susceptible to developing a cardiovascular disorder if the morphological feature of the population of circulating endothelial cells deviates from the control or standard by at least a predetermined amount.

Disclosed herein, in certain embodiments, are methods of predicting an individual's response to a treatment regimen for a cardiovascular disorder, comprising: (a) comparing a morphological feature of: (i) a population of circulating endothelial cells isolated from a blood sample obtained from the individual, wherein the blood sample comprises cells selected from circulating endothelial cells and circulating non-endothelial cells; and (ii) control or standard; and (b) predicting the individual will be responsive to a treatment regimen for a cardiovascular disorder if the morphological feature of the population of circulating endothelial cells deviates from the control or standard by at least a predetermined amount.

Disclosed herein, in certain embodiments, are methods of analyzing a cardiovascular disorder comprising: (a) identifying a morphological feature of: a circulating endothelial cell (CEC) isolated from a blood sample obtained from the individual, wherein the blood sample comprises cells selected from CECs and circulating non-endothelial cells; (b) determining whether the morphological feature of a CEC from an individual with a cardiovascular disorder is one that relates to a cardiovascular disorder; and (c) providing diagnostic or prognostic information about the cardiovascular disorder based on the identification; wherein said morphological feature of a CEC from an individual with a cardiovascular disorder is selected from: a large cellular area relative to CECs from a control or standard , a large nuclear area relative to CECs from a control or standard, a large ratio of cellular area to nuclear area relative to CECs from a control or standard, cell shape, nuclei shape, and the presence of more than one nuclei.

Further disclosed herein, in certain embodiments, are isolated circulating endothelial cells comprising one morphological feature that deviates from the morphological features of a control or standard. Also, disclosed herein are compositions comprising isolated CECs.

Further disclosed herein, in certain embodiments, are systems comprising isolated circulating endothelial cells comprising one morphological feature that deviates from the morphological features of a control or standard, and either (i) an analytical tool that allows analysis of the aforementioned morphological feature, or (ii) the output from an analytical tool that allows a health care provider to analyze the aforementioned morphological feature.

### Certain Terminology

The terms "individual," "individual," or "subject" are used interchangeably. As used herein, they mean any mammal (i.e. species of any orders, families, and genus within the taxonomic classification animalia: chordata: vertebrata: mammalia). In some embodiments, the mammal is a human. In some embodiments, the mammal is a non-human. None of the terms require or are limited to situation characterized by the supervision (e.g. constant or intermittent) of a medical professional (e.g. a doctor, a registered nurse, a nurse practitioner, a physician's assistant, an orderly, or a hospice worker).

The term "healthy individual" means an individual without a cardiovascular disorder. It does not require than the individual be free from any and all diseases, disorders, or conditions.

The terms "treat," "treating" or "treatment," and other grammatical equivalents as used herein, include alleviating, inhibiting or reducing symptoms, reducing or inhibiting severity of, reducing incidence of, prophylactic treatment of, reducing or inhibiting recurrence of, preventing, delaying onset of, delaying recurrence of, abating or ameliorating a disease or condition symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. The terms further include achieving a therapeutic benefit. By therapeutic benefit is meant eradication or amelioration of the underlying disorder being treated, and/or the eradication or amelioration of one or more of the physiological symptoms associated with the underlying disorder such that an improvement is observed in the individual.

The terms "prevent," "preventing" or "prevention," and other grammatical equivalents as used herein, include preventing additional symptoms, preventing the underlying metabolic causes of symptoms, inhibiting the disease or condition, e.g., arresting the development of the disease or condition and are intended to include prophylaxis. The terms further include achieving a prophylactic benefit. For prophylactic benefit, the compositions are optionally administered to an individual at risk of developing a particular disease, to an individual reporting one or more of the physiological symptoms of a disease, or to an individual at risk of reoccurrence of the disease.

The terms "administer," "administering", "administration," and the like, as used herein, refer to the methods that may be used to enable delivery of agents or compositions to the desired site of biological action. These methods include, but are not limited to oral routes, intraduodenal routes, parenteral injection (including intravenous, subcutaneous, intraperitoneal, intramuscular, intravascular or infusion), topical and rectal administration. Administration techniques that are optionally employed with the agents and methods described herein, include e.g., as discussed in Goodman and Gilman, The Pharmacological Basis of Therapeutics, current ed.; Pergamon; and Remington's, Pharmaceutical Sciences (current edition), Mack Publishing Co., Easton, Pa. In certain embodiments, the agents and compositions described herein are administered orally.

As used herein, the terms "antibody" and "antibodies" refer to monoclonal antibodies, polyclonal antibodies, bi-specific antibodies, multispecific antibodies, grafted antibodies, human antibodies, humanized antibodies, synthetic antibodies, chimeric antibodies, camelized antibodies, single-chain Fvs (scFv), single chain antibodies, Fab fragments, F(ab') fragments, disulfide-linked Fvs (sdFv), intrabodies, and anti-idiotypic (anti-Id) antibodies and antigen-binding fragments of any of the above. In particular, antibodies include immunoglobulin molecules and immunologically active fragments of immunoglobulin molecules, i.e., molecules that contain an antigen binding site. Immunoglobulin molecules are of any type (e.g., IgG, IgE, IgM, IgD, IgA and IgY), class (e.g., IgG₁, IgG ₂, IgG ₃, IgG ₄, IgA ₁ and IgA ₂) or subclass. The terms "antibody" and immunoglobulin are used interchangeably in the broadest sense. In some embodiments an antibody is part of a larger fusion molecule, formed by covalent or non-covalent association of the antibody with one or more other proteins or peptides..

The phrase "specifically binds" when referring to the interaction between an antibody or other binding molecule and a protein or polypeptide or epitope, typically refers to an antibody or other binding molecule that recognizes and detectably binds with high affinity to the target of interest. Preferably, under designated or physiological conditions, the specified antibodies or binding molecules bind to a particular polypeptide, protein or epitope yet does not bind in a significant or undesirable amount to other molecules present in a sample. In other words the specified antibody or binding molecule does not undesirably cross-react with non-target antigens and/or epitopes. Further, in some embodiments, an antibody that specifically binds, binds through the variable domain or the constant domain of the antibody. For the antibody that specifically binds through its variable domain, it is not aggregated, i.e., is monomeric. A variety of immunoassay formats are used to select antibodies or other binding molecule that are immunoreactive with a particular polypeptide and have a desired specificity. For example, solid-phase ELISA immunoassays, BIAcore, flow cytometry and radioimmunoassays are used to select monoclonal antibodies having a desired immunoreactivity and specificity. See, Harlow, 1988, ANTIBODIES, A LABORATORY MANUAL, Cold Spring Harbor Publications, New York (hereinafter, "Harlow"), for a description of immunoassay formats and conditions that are used to determine or assess immunoreactivity and specificity. "Selective binding", "selectivity", and the like refer the preference of a antibody to interact with one molecule as compared to another. Preferably, interactions between antibodies, particularly modulators, and proteins are both specific and selective. Note that in some embodiments a small antibody is designed to "specifically bind" and "selectively bind" two distinct, yet similar targets without binding to other undesirable targets.

The term "cardiovascular disorder" means a disease, disorder, or undesired condition of the cardiovascular system. In some embodiments, the cardiovascular disorder is atherosclerosis, myocardial infarction, ischemic stroke, plaque rupture, plaque erosion, ischemia of the heart, reperfusion injury to the heart, thrombosis, angina (e.g., stable or unstable angina), ST-segment elevation myocardial infarction (STEMI), non-ST-segment elevation myocardial infarction (NSTEMI), or a combination thereof.

The term "morphological feature" means a feature of a cell that is associated with the form and/or structure of cell. Morphological features of cells include, but are not limited to, cell shape, nuclear shape, cellular area, nuclear area, ratio of cellular area to nuclear area, and number of nuclei.

The term "circulating endothelial cell" means an endothelial cell that has become detached from the wall of a blood vessel and circulates in blood. Endothelial cells lining the interior surfaces of the heart chambers are called endocardial cells

The terms "control" or "standard" are used interchangeably. The mean a CEC standard of comparison against which a CEC isolated from an individual with a cardiovascular disorder is compared. In some embodiments, the control is a CEC or plurality of CECs derived from a healthy individual. In some embodiments, the control is a CEC or plurality of CECs derived from an individual who has or has had a cardiovascular disorder of interest. In some embodiments, the control is a CEC or a plurality of CECs. In some embodiments, the control is a measurement associated with a CEC or plurality of CECs (e.g., a morphological feature of a CEC, such as cellular area, cellular shape, nuclear area, nuclear shape, number of nuclei, or ratio of cellular area to nuclear area; or the concentration of CECs in blood).In some embodiments, the control or standard is an average CEC measurement derived from measurements obtained from a plurality of individuals.

The terms "diagnostic information" means information related to the characteristics of a disease, disorder, or condition that enable identification of the disease, disorder, or condition. In some embodiments, the diagnostic information is the morphological features of a CEC or plurality of CECs, or the concentration of CECs in blood.

The term "prognostic information" means information that enables one to predict a future event. Prognostic information may predict the likely outcome of a disease, disorder, or condition. Alternatively, prognostic information may predict how a disease, disorder, or condition will respond to a treatment. In some embodiments, the prognostic information is the morphological features of a CEC or plurality of CECs, or the concentration of CECs in blood.

### Cardiovascular Disorders and Plaque Rupture

Disclosed herein, in certain embodiments, are methods of diagnosing or predicting plaque rupture in an individual in need thereof. Further disclosed herein, in certain embodiments, are methods of analyzing, diagnosing or predicting cardiovascular disorders in an individual in need thereof. Additionally, disclosed herein in certain embodiments, are methods of predicting an individual's response to a cardiovascular disorder treatment regimen.

In some individuals, the individual is diagnosed with a cardiovascular disorder. In some embodiments, the individual is suspected of having a cardiovascular disorder. In some embodiments, the individual has no symptoms of a cardiovascular disorder.

In some embodiments, the individual exhibits a symptom selected from: chest pain or discomfort; pain in the arms, neck, jaw, shoulder or back accompanying chest pain; nausea; fatigue; shortness of breath; sweating; dizziness; numbness or weakness of the face, arm or leg, especially on one side of the body; confusion, trouble speaking or understanding; trouble seeing in one or both eyes; trouble walking, dizziness, loss of balance or coordination; severe headache with no known cause or any combination thereof.

In some embodiments, the cardiovascular disorder is atherosclerosis, myocardial infarction, ischemic stroke, plaque rupture, plaque erosion, ischemia of the heart, reperfusion injury to the heart, thrombosis, angina (e.g., stable or unstable angina), ST-segment elevation myocardial infarction (STEMI), non-ST-segment elevation myocardial infarction (NSTEMI), or a combination thereof.

As used herein, the term "therosclerosis" refers to the hardening and thickening of an arterial wall due to the deposition of lipids and the resulting inflammation and white blood cell response. Left untreated an atherosclerostic plaque may eventually result in partial or complete occlusion of the artery.

The terms "Myocardial infarction and heart attack are used interchangeably. As used herein, both terms refer to an interruption in the blood supply to the heart. Interruption in the blood supply to the heart often results from the occlusion of a coronary artery by a ruptured atherosclerotic plaque. Further, myocardial infarctions often lead to scarring of myocardial tissue which may result in a slowing of the conduction of electrical impulses. Differences in conduction velocity between scarred and unscarred tissue often leads to ventricular fibrillation or ventricular tachycardia.

As used herein, the term "ischemic stroke" refers to a loss of brain function (e.g., necrosis of brain tissue) resulting from (partially or fully) a disturbance in blood supply (e.g., ischemia) to the brain. In certain instances, a stroke results from (partially or fully) a plaque rupture, thrombosis or an embolism.

As used herein, the term "thrombosis" refers to the formation of a blood clot. When a blood clot forms in a vein it is referred to as venous thrombosis. When the blood clot forms in an artery it is referred to as arterial thrombosis. If the blood clot (or a piece thereof) is transported (i.e., an embolism) to the lungs a pulmonary embolism may develop.

As used herein, the term "angina" refers to severe chest pain resulting from ischemia of the heart (e.g., due to occlusion of a blood vessel or artery following expansion of an atherosclerotic plaque). Stable angina chest refers to chest pains induced by an activity (e.g., running, walking) with minimal or non-existent symptoms at rest. Symptoms typically abate several minutes following cessation of the activity and resume when activity resumes. Unstable angina refers to chest pains developing during rest or sleep and usually lasting >10 min. The associated chest pains are severe and newly developed.

As used herein, the term "STEMI" means a ST segment elevation myocardial infarction (e.g., a myocardial infarction resulting in ST segment elevation on an ECG). STEMI often result from the complete occlusion of a coronary artery. The resulting ST segment indicates that a significant portion of the heart muscle is damaged or dying. About 45% of all myocardial infarctions are categorized as STEMI.

As used herein, the term "NSTEMI" means a non-ST segment elevation myocardial infarction (e.g., a myocardial infarction that does not result in ST segment elevation on an ECG).

In a NSTEMI, the coronary artery is only partially occluded and thus only a portion of the heart muscle is damaged.

As used herein, the term "stenosis" means the narrowing of a blood vessel. Stenosis often results from the expansion of an atherosclerotic plaque for the rupture of a plaque and the subsequent formation of a thrombus.

As used herein, the term "ischemia" means a restriction in blood supply. Ischemia often results occlusion of a blood vessel or artery following expansion of an atherosclerotic plaque (e.g., due to the formation of a thrombus following rupture of the plaque).

### Pathobiology

The heart is a myogenic muscular organ found in animals with a circulatory system. In mammals, the beating of the right side of the heart results in the movement of de-oxygenated blood through the right atrium and into the lungs where carbon dioxide is removed and the blood is oxygenated. The beating of the left side moves the oxygenated blood, via the arteries, from the lungs to heart, where it is then distributes to the rest of the body.

The three major coronary arteries (Left Anterior Descending (LAD), Circumflex (Circ) and Right Coronary Artery (RCA)) and their respective branches each supply a designated portion of the heart. The LAD supplies blood to the front (anterior) portion of the heart and the septum (muscle partition that separates the Left Ventricle (LV) and Right Ventricle (RV)). The Circ supplies the back (posterior) portion of the LV. The RCA supplies the bottom (inferior) portion of the ventricle and also the RV in 90% of cases. In the other 10%, the Circ sends a branch to the inferior wall of the LV.

Occasionally, blood flow through the arteries is reduced (e.g., due to a coronary artery blockage). A temporary decrease in blood flow may result in chest discomfort. A persistent decrease may result in permanent muscle damage, a heart attack, or stroke.

Atherosclerosis is the most common cause of coronary artery blockage. Atherosclerosis is characterized by a thickening of the arterial wall. Atherosclerosis often begins with accumulation of low-density lipoprotein molecules (LDL) in the arterial wall. Eventually, the LDL molecules become oxidized by free radicals (e.g., ROS). When contacted with the arterial wall, the oxidized LDL damages the wall. The body responds by attempting to repair this damage with macrophages and T-lymphocytes. These white blood cells engulf oxidized low-density lipoproteins (LDLs) by endocytosis. The oxidized LDL accumulates in the white blood cells which are transformed into foam cells. Foam cells accumulate around the build-up and eventually rupture. Rupture of a foam cells results in the deposition of the oxidized-LDL back onto the arterial wall. This then triggers the migration of additional white blood cells to the developing plaque. Eventually, the area around the plaque becomes inflamed. This causes muscle cells to enlarge and form a hard cover over the affected area. This hard cover causes a narrowing of the artery, which may reduce the blood flow and increase blood pressure.

Atherosclerosis is often asymptomatic until the plaque ruptures, usually at the site of thinner/weaker fibrous caps that have become "unstable". If the fibrous cap separating a soft plaque from the bloodstream ruptures, tissue fragments are exposed and released. These tissue fragments contain collagen and tissue factor which activate platelets and activate coagulation. The result is the formation of a thrombus overlying the plaque, which further obstructs blood flow. The formation of a thrombus may occur within a matter of hours.

If the plaque is upstream of the heart and there is an incomplete blockage, the resulting decrease in blood flow to the heart may result in severe and prolonged chest pain (e.g., unstable angina). In some embodiments, the cardiovascular disorder is unstable angina. Alternatively, if there is a complete blockage, the heart is deprived of oxygen and an MI develops. In some embodiments, the cardiovascular disorder is myocardial infarction.

If the rupture is upstream of the brain, the brain is starved for oxygen and an ischemic stroke develops. In some embodiments, the cardiovascular disorder is ischemic stroke.

### CECs

The endothelium is the layer of cells that lines the interior surface of blood vessels, forming an interface between circulating blood in the lumen and the rest of the vessel wall. Endothelial cells line the entire circulatory system. In certain instances, endothelial cells become detached from the vessel wall, resulting in circulating endothelial cells.

### Circulating endothelial cells are found in healthy individuals.

The lack of normal endothelial integrity (and thus, the presence of circulating endothelial cells) has been previously identified as a marker for susceptibility to atherosclerotic plaque rupture as well as other arterial, non-atherosclerotic vessel wall rupture or fissure events. Thus, in some embodiments, the presence of circulating endothelial cells is indicative of susceptibility to plaque rupture or other arterial, non-atherosclerotic vessel wall rupture or fissure events. Further, in some embodiments, an increase in the concentration of circulating endothelial cells as compared to the concentration seen in healthy individuals (or, a predetermined control) is indicative of an upcoming to plaque rupture or other arterial, non-atherosclerotic vessel wall rupture or fissure events

### Analysis of CECs

CECs from individuals with a cardiovascular disorder (e.g., individuals at risk for or suffering from a plaque rupture or other arterial, non-atherosclerotic vessel wall rupture or fissure events) show measurable differences in morphological parameters, namely, cell and nuclear areas, ratio of cellular/nuclear area, and association with multiple nuclei, when compared to healthy individuals or predetermined controls.

Disclosed herein, in certain embodiments, are methods of analyzing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard. Disclosed herein, in certain embodiments, are methods of diagnosing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard; and (c) diagnosing a cardiovascular disorder in the individual if the morphological feature(s) of the population of circulating endothelial cells (i) deviates from the control or standard from a healthy individual or population of healthy individuals, or (ii) matches the control or standard from an individual or population of individuals who have or have had the cardiovascular disease. Disclosed herein, in certain embodiments, are methods of predicting whether an individual is susceptible to developing a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the susceptibility of the individual for developing a cardiovascular disorder based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. Disclosed herein, in certain embodiments, are methods of predicting an individual's response to a treatment regimen for a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the individual's response to a treatment regimen based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. Disclosed herein, in certain embodiments, are methods of prescribing a treatment regimen for a cardiovascular disorder to an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) prescribing a treatment regimen based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease.

In some embodiments, the methods disclosed herein comprise comparing at least one morphological feature of a CEC from an individual to a control or standard. In some embodiments, CECs in individuals suffering from a cardiovascular disorder (e.g., individuals at risk for or suffering from a plaque rupture or other arterial, non-atherosclerotic vessel wall rupture or fissure events) have discrete morphological signatures indicative of abnormal endothelial biology. In some embodiments, CECs in individuals suffering from a cardiovascular disorder (e.g., individuals at risk for or suffering from a plaque rupture or other arterial, non-atherosclerotic vessel wall rupture or fissure events) originate from older, and thus larger, cells in the arterial endothelium. In some embodiments, CECs in individuals suffering from a cardiovascular disorder (e.g., individuals at risk for or suffering from a plaque rupture or other arterial, non-atherosclerotic vessel wall rupture or fissure events) release singly or as multicellular groups or sheets from injured regions during plaque rupture. In some embodiments, CECs in individuals suffering from a cardiovascular disorder (e.g., individuals at risk for or suffering from a plaque rupture or other arterial, non-atherosclerotic vessel wall rupture or fissure events) originate from abnormally aggregated (e.g., aggregated during their passage through the circulation) injured or disturbed cells. In some embodiments, CECs in healthy individuals result from infrequent release of younger and thus smaller ECs from regions of remodeling or proliferating vasculature.

In some embodiments, the methods disclosed herein comprise comparing at least two morphological features of a CEC from an individual to a control or standard. In some embodiments, the methods disclosed herein comprise comparing at least three morphological features of a CEC from an individual to a control or standard. In some embodiments, the methods disclosed herein comprise comparing at least four morphological features of a CEC from an individual to a control or standard. In some embodiments, the methods disclosed herein comprise comparing at least five morphological features of a CEC from an individual to a control or standard. In some embodiments, the methods disclosed herein comprise comparing at least six morphological features of a CEC from an individual to a control or standard.

In some embodiments, the morphological feature of a CEC from an individual with a cardiovascular disorder is: cellular area; nuclear area; ratio of cellular area to nuclear area; cell shape; nuclei shape; number of nuclei; or a combination thereof. In some embodiments, the morphological feature of a CEC from an individual with a cardiovascular disorder is cellular area. In some embodiments, the morphological feature of a CEC from an individual with a cardiovascular disorder is nuclear area. In some embodiments, the morphological feature of a CEC from an individual with a cardiovascular disorder is ratio of cellular area to nuclear area. In some embodiments, the morphological feature of a CEC from an individual with a cardiovascular disorder is cell shape. In some embodiments, the morphological feature of a CEC from an individual with a cardiovascular disorder is nuclei shape. In some embodiments, the morphological feature of a CEC from an individual with a cardiovascular disorder is number of nuclei.

In some embodiments, the morphological feature of a CEC from an individual with a cardiovascular disorder is: a large cellular area relative to CECs from a control or standard; a large nuclear area relative to CECs from a control or standard; or a large ratio of cellular area to nuclear area relative to CECs from a control or standard; or a combination thereof In some embodiments, the morphological feature of a CEC from an individual with a cardiovascular disorder is a large cellular area relative to CECs from a control or standard. In some embodiments, the morphological feature of a CEC from an individual with a cardiovascular disorder is a large nuclear area relative to CECs from a control or standard. In some embodiments, the morphological feature of a CEC from an individual with a cardiovascular disorder is a large ratio of cellular area to nuclear area relative to CECs from a control or standard; or a combination thereof

CECs from an individual without a cardiovascular disorder tend to be small, and elongated in shape. In some embodiments, CECs from an individual with a cardiovascular disorder tend to be heterogeneous - some are small and some are large. In some embodiments, the CECs from individuals with cardiovascular disorders tend to be abnormally shaped. Additionally, in some embodiments, the nucleus of CECs from individuals with cardiovascular disorders tend to be larger than normal and abnormally shaped.

In some embodiments, the morphological feature of a CEC from an individual with a cardiovascular disorder is a cellular area that is at least 5%, 10%, 15%, 20%, 25%, 30%, 40%, 45%, 50%, 60%, 70%, 80%, 90% or 100% greater than the average cellular area of a CEC from a control or standard. In some embodiments, the morphological feature of a CEC from an individual with a cardiovascular disorder is a cellular area that is between about 30% and about 50% greater than the average cellular area of a CEC from a control or standard. In some embodiments, the morphological feature of a CEC from an individual with a cardiovascular disorder is a cellular area that is between about 30% greater than the average cellular area of a CEC from a control or standard. In some embodiments, the morphological feature of a CEC from an individual with a cardiovascular disorder is a cellular area that is between about 35% greater than the average cellular area of a CEC from a control or standard. In some embodiments, the morphological feature of a CEC from an individual with a cardiovascular disorder is a cellular area that is between about 40% greater than the average cellular area of a CEC from a control or standard. In some embodiments, the morphological feature of a CEC from an individual with a cardiovascular disorder is a cellular area that is between about 45% greater than the average cellular area of a CEC from a control or standard. In some embodiments, the morphological feature of a CEC from an individual with a cardiovascular disorder is a cellular area that is between about 50% greater than the average cellular area of a CEC from a control or standard.

The average cellular area of a CEC in an individual without a cardiovascular disorder is about about100 mm². Thus, in some embodiments, the cellular area of a CEC indicative of a cardiovascular disorder is greater than about 100 µm², about 150 µm², about 200 µm², about 210 µm², about 220 µm², about 230 µm², about 240 µm², about 250 µm², about 260 µm², about 270 µm², about 280 µm², about 290 µm², or about 300 µm². In some embodiments, the cellular area of a CEC indicative of a cardiovascular disorder is greater than about 100 µm². In some embodiments, the cellular area of a CEC indicative of a cardiovascular disorder is greater than about 150 µm². In some embodiments, the cellular area of a CEC indicative of a cardiovascular disorder is greater than about 200 µm². In some embodiments, the cellular area of a CEC indicative of a cardiovascular disorder is about 200 µm². In some embodiments, the cellular area of a CEC indicative of a cardiovascular disorder is greater than about 250 µm². In some embodiments, the cellular area of a CEC indicative of a cardiovascular disorder is about 250 µm². In some embodiments, the cellular area of a CEC indicative of a cardiovascular disorder is greater than about 255 µm². In some embodiments, the cellular area of a CEC indicative of a cardiovascular disorder is about 255 µm². In some embodiments, the cellular area of a CEC indicative of a cardiovascular disorder is greater than about 260 µm². In some embodiments, the cellular area of a CEC indicative of a cardiovascular disorder is about 260 µm². In some embodiments, the cellular area of a CEC indicative of a cardiovascular disorder is greater than about 265 µm². In some embodiments, the cellular area of a CEC indicative of a cardiovascular disorder is about 265 µm². In some embodiments, the cellular area of a CEC indicative of a cardiovascular disorder is about 270 µm².

In some embodiments, the morphological feature of a CEC from an individual with a cardiovascular disorder is a nuclear area that is at least 5%, 10%, 15%, 20%, 25%, 30%, 40%, 45%, 50%, 60%, 70%, 80%, 90% or 100% greater than the average cellular area of a CEC from a control or standard. In some embodiments, the morphological feature of a CEC from an individual with a cardiovascular disorder is a nuclear area that is at least 30% greater than the average cellular area of a CEC from a control or standard. In some embodiments, the morphological feature of a CEC from an individual with a cardiovascular disorder is a nuclear area that is at least 35% greater than the average cellular area of a CEC from a control or standard. In some embodiments, the morphological feature of a CEC from an individual with a cardiovascular disorder is a nuclear area that is at least 40% greater than the average cellular area of a CEC from a control or standard. In some embodiments, the morphological feature of a CEC from an individual with a cardiovascular disorder is a nuclear area that is at least 45% greater than the average cellular area of a CEC from a control or standard. In some embodiments, the morphological feature of a CEC from an individual with a cardiovascular disorder is a nuclear area that is at least 50% greater than the average cellular area of a CEC from a control or standard. In some embodiments, the morphological feature of a CEC from an individual with a cardiovascular disorder is a nuclear area that is between about 30% and about 50% greater than the average cellular area of a CEC from a control or standard.

The average cellular area of a CEC in an individual without a cardiovascular disorder is between about 30 and about 40 mm². In some embodiments, the nuclear area is greater than about 30 µm², about 31 µm², about 32 µm², about 33 µm², about 34 µm², about 35 µm², about 36 µm², about 37 µm², about 38 µm², about 39 µm², about 40 µm², about 50 µm², about 60 µm², about 70 µm², about 80 µm², about 90 µm², or about 100 µm². In some embodiments, the nuclear area is greater than 30 µm². In some embodiments, the nuclear area is greater than about 35 µm². In some embodiments, the nuclear area is about 35 µm². In some embodiments, the nuclear area is greater than about 40 µm². In some embodiments, the nuclear area is about 40 µm². In some embodiments, the nuclear area is greater than about 45 µm². In some embodiments, the nuclear area is about 45 µm². In some embodiments, the nuclear area is greater than about 50 µm². In some embodiments, the nuclear area is about 50 µm². In some embodiments, the nuclear area is greater than about 55 µm². In some embodiments, the nuclear area is about 55 µm². In some embodiments, the nuclear area is greater than about 60 µm². In some embodiments, the nuclear area is about 60 µm². In some embodiments, the nuclear area of a CEC indicative of a cardiovascular disorder is about 65 µm².

CECs from individuals without a cardiovascular disorder may have two nuclei associated with one cell body, which may represent a cluster of two attached cells, or one cell with two nuclei. In some embodiments, CECs from individuals with cardiovascular disorders often contain several nuclei associated with one continuous cell body. These bodies may be a single cell or an aggregate of multiple cells. In some embodiments, the morphological feature of a CEC from an individual with a cardiovascular disorder is the presence of more than one nuclei. In some embodiments, the morphological feature of a CEC from an individual with a cardiovascular disorder is the presence of two nuclei. In some embodiments, the morphological feature of a CEC from an individual with a cardiovascular disorder is the presence of three nuclei. In some embodiments, the morphological feature of a CEC from an individual with a cardiovascular disorder is the presence of four nuclei. In some embodiments, the morphological feature of a CEC from an individual with a cardiovascular disorder is the presence of five nuclei. In some embodiments, the morphological feature of a CEC from an individual with a cardiovascular disorder is the presence of six nuclei. In some embodiments, the morphological feature of a CEC from an individual with a cardiovascular disorder is the presence of seven nuclei. In some embodiments, the morphological feature of a CEC from an individual with a cardiovascular disorder is the presence of eight nuclei. In some embodiments, the morphological feature of a CEC from an individual with a cardiovascular disorder is the presence of nine nuclei. In some embodiments, the morphological feature of a CEC from an individual with a cardiovascular disorder is the presence of ten nuclei. In some embodiments, the morphological feature of a CEC from an individual with a cardiovascular disorder is the presence of at least 10% more nuclei than the average number of nuclei of a CEC from a control or standard.

In some embodiments, there is no statistically significant difference in cellular and nuclear sizes, ratios, or presence of multiple nuclei between healthy individuals. In some embodiments, there are no statistically significant differences between ST segment elevation myocardial infarction (STEMI) and Non-ST segment elevation myocardial infarction (NSTEMI) CECs.

In some embodiments, the methods disclosed herein further comprise comparing: (a) the concentration of the population of circulating endothelial cells isolated from the blood sample; and (b) the concentration of control or standard. In some embodiments, the concentration of CECs in an individual with a cardiovascular disorder is at least 50%, 100%, 150%, 200%, 250%, 300%, 350%, 400%, 450%, 500%, 550%, 600%, 650%, or 700% greater than the concentration of CECs found in a healthy individual or a predetermined control. In some embodiments, the concentration of CECs in an individual with a cardiovascular disorder is between about 200% and about 400% greater than the concentration of CECs found in a healthy individual or a predetermined control.

In some embodiments, comparing a morphological feature of the population of circulating endothelial cells (or, single CECs) comprises analyzing magnified images (e.g., fluorescent images) of the population of isolated circulating endothelial cells obtained by use of any suitable microscope. In some embodiments, comparing a morphological feature of the population of circulating endothelial cells (or, single CECs) comprises analyzing magnified fluorescent images of the population of isolated circulating endothelial cells. In some embodiments, the microscope has magnifying power that is sufficient to visualize a morphological feature of a single CEC in the population of CECs.

In some embodiments the magnified images of the CECs are stored in the electronic memory of a computer-processing device associated with the microscope.

In some embodiments, the methods disclosed herein comprise any suitable software module, executed by a computer-processing device, analyzing at least one morphological feature of the circulating endothelial cells. In some embodiments, the methods disclosed herein comprise any suitable computer software module, executed by a computer-processing device, comparing at least one morphological feature of the circulating endothelial cells to a control or standard, wherein the morphological feature of a CEC from an individual with a cardiovascular disorder is cellular area; nuclear area; ratio of cellular area to nuclear area; cell shape; nuclei shape; number of nuclei; or a combination thereof. In some embodiments, comparing a morphological feature of the population of circulating endothelial cells comprises any suitable software module, executed by a computer-processing device, analyzing at least one morphological feature of the circulating endothelial cells, wherein the morphological feature of a CEC from an individual with a cardiovascular disorder is a large cellular area relative to CECs from a control or standard; a large nuclear area relative to CECs from a control or standard; or a large ratio of cellular area to nuclear area relative to CECs from a control or standard; or a combination thereof In some embodiments, the methods disclosed herein comprise a software module executed by the computer-processing device determining concentration of the population of circulating endothelial cells isolated from the blood sample. In some embodiments, the methods disclosed herein comprise a software module executed by the computer-processing device comparing the concentration of the population of circulating endothelial cells to the concentration of control or standard. In some embodiments, the methods disclosed herein comprise a software module executed by the computer-processing device transmitting an analysis of the morphological feature of the population of CECs to the individual or a medical professional treating the individual. In some embodiments, the methods disclosed herein comprise a software module executed by the computer-processing device transmitting a diagnosis or prognosis to the individual or a medical professional treating the individual.

The methods disclosed herein are executed by any suitable systems. In some embodiments, the systems comprise (a) a population of circulating endothelial cells (CEC) isolated from a blood sample obtained from an individual, wherein the blood sample comprises cells selected from CECs and circulating non-endothelial cells; and (b) a microscope having sufficient magnifying power to visualize a morphological feature of a single CEC in the population of CECs; wherein the morphological feature is selected from: cellular area, nuclear area, ratio of cellular area to nuclear area, cell shape, nuclei shape, number of nuclei, number of circulating endothelial cells, or a combination thereof. In some embodiments, the systems further comprise electronic memory for capturing and storing a magnified image of the population of CECs or a single CEC in the population of CECs. In some embodiments, the systems further comprise a computer-processing device, optionally connected to a computer network. In some embodiments, the systems further comprise a software module executed by the computer-processing device to analyze a morphological feature of the population of circulating endothelial cells (CEC). In some embodiments, the systems further comprise a software module executed by the computer-processing device to compare the morphological feature of the population of circulating endothelial cells (CEC) to a standard or control. In some embodiments, the systems further comprise a software module executed by the computer-processing device to determine concentration of the population of circulating endothelial cells isolated from the blood sample. In some embodiments, the systems further comprise a software module executed by the computer-processing device to compare the concentration of the population of circulating endothelial cells to the concentration of control or standard. In some embodiments, the systems further comprise a software module executed by the computer-processing device to transmit an analysis of the morphological feature of the population of CECs to the individual or a medical professional treating the individual. In some embodiments, the systems further comprise a software module executed by the computer-processing device to transmit a diagnosis or prognosis to the individual or a medical professional treating the individual.

In some embodiments, the systems further comprise a machine to isolate the population of circulating endothelial cells from the blood sample. In some embodiments, the systems further comprise a label that specifically binds to circulating endothelial cells, a label that does not bind to circulating endothelial cells, or a combination thereof. In some embodiments, the systems further comprise a label that specifically binds to CD 146, a label that specifically binds to CD105, a label that specifically binds to CD45, a label that specifically binds to CD34, a label that specifically binds to CD31, a label that specifically binds to VEGF2, a label that specifically binds to CD 144, a label that specifically binds to CD106, or a combination thereof In some embodiments, the systems further comprise anti-CD 146 antibodies, anti-CD 105 antibodies, anti-CD45 antibodies, anti CD34 antibodies, anti-CD31 antibodies, anti-VEGF2 antibodies, anti-CD 144 antibodies, anti-CD106 antibodies, or a combination thereof In some embodiments, the antibodies are labeled. In some embodiments, the antibodies are labeled with a fluorescent moiety or a magnetic moiety.

### Isolation of CECs

Current methods of isolating CECs produce variability in the results. This variability stems primarily from the use of highly divergent CEC isolation methods and variable immunophenotypic defmitions of CECs.

Disclosed herein, in certain embodiments, are methods of analyzing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard. In some embodiments, the methods disclosed herein further comprise methods of isolating CECs. In some embodiments, the methods further comprise isolating CECs using positive and negative selection criteria.

Disclosed herein, in certain embodiments, are methods of diagnosing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard; and (c) diagnosing a cardiovascular disorder in the individual if the morphological feature(s) of the population of circulating endothelial cells (i) deviates from the control or standard from a healthy individual or population of healthy individuals, or (ii) matches the control or standard from an individual or population of individuals who have or have had the cardiovascular disease. In some embodiments, the methods disclosed herein further comprise methods of isolating CECs. In some embodiments, the methods further comprise isolating CECs using positive and negative selection criteria.

Disclosed herein, in certain embodiments, are methods of predicting whether an individual is susceptible to developing a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the susceptibility of the individual for developing a cardiovascular disorder based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. In some embodiments, the methods disclosed herein further comprise methods of isolating CECs. In some embodiments, the methods further comprise isolating CECs using positive and negative selection criteria.

Disclosed herein, in certain embodiments, are methods of predicting an individual's response to a treatment regimen for a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the individual's response to a treatment regimen based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. In some embodiments, the methods disclosed herein further comprise methods of isolating CECs. In some embodiments, the methods further comprise isolating CECs using positive and negative selection criteria.

Disclosed herein, in certain embodiments, are methods of prescribing a treatment regimen for a cardiovascular disorder to an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) prescribing a treatment regimen based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. In some embodiments, the methods disclosed herein further comprise methods of isolating CECs. In some embodiments, the methods further comprise isolating CECs using positive and negative selection criteria.

In some embodiments, the methods disclosed herein further comprise contacting the blood sample with a label that specifically binds to circulating endothelial cells, a label that does not bind to circulating endothelial cells, or a combination thereof. In some embodiments, the methods disclosed herein further comprise contacting a blood sample with a label that specifically binds to CD 146, a label that specifically binds to CD105, a label that specifically binds to CD45, a label that specifically binds to CD34, a label that specifically binds to CD31, a label that specifically binds to VEGF2, a label that specifically binds to CD 144, a label that specifically binds to CD106, or a combination thereof.

In some embodiments, the methods disclosed herein further comprise contacting the blood sample with anti-CD 146 antibodies, anti-CD105 antibodies, anti-CD45 antibodies, anti-CD34 antibodies, anti-CD31 antibodies, anti-VEGF2 antibodies, anti-CD144 antibodies, anti-CD106 antibodies or a combination thereof.

In some embodiments, the antibodies are labeled with any suitable label. In some embodiments, the antibodies are labeled with a fluorescent moiety. In some embodiments, the antibodies are labeled with a magnetic moiety.

In some embodiments, isolating the population of circulating endothelial cells from the blood sample comprises: (a) contacting the blood sample with anti-CD146 antibodies; and (b) isolating the cells to which the anti-CD146 antibodies specifically bind, to generate a population of CD146+ cells. In some embodiments, isolating the population of circulating endothelial cells from the blood sample comprises: (a) contacting the blood sample with anti-CD146 antibodies, wherein the antibodies are magnetically labeled; and (b) isolating the cells to which the anti-CD 146 antibodies specifically bind, to generate a population of CD146+ cells. In some embodiments, isolating the population of circulating endothelial cells from the blood sample comprises: (a) contacting the blood sample with anti-CD146 antibodies, wherein the antibodies are fluorescently labeled; and (b) isolating the cells to which the anti-CD146 antibodies specifically bind, to generate a population of CD146+ cells.

In some embodiments, the methods disclosed herein further comprise contacting the population of CD146+ cells with anti-CD45 antibodies, anti-CD105 antibodies, DAPI, or a combination thereof. In some embodiments, the methods disclosed herein further comprise contacting the population of CD146+ cells with anti-CD45 antibodies that have been labeled, anti-CD105 antibodies that have been labeled, anti-CD34 antibodies that have been labeled, anti-CD31 antibodies that have been labeled, anti-VEGFR2 antibodies that have been labeled, anti CD-144 antibodies that have been labeled, anti-CD106 antibodies that have been labeled, DAPI, or a combination thereof. In some embodiments, the methods disclosed herein further comprise contacting the population of CD146+ cells with anti-CD45 antibodies that have been magnetically labeled, anti-CD105 antibodies that have been magnetically labeled, anti-CD34 antibodies that have been magnetically labeled, anti-CD31 antibodies that have been magnetically labeled, anti-VEGFR2 antibodies that have been magnetically labeled, anti CD-144 antibodies that have been magnetically labeled, anti-CD106 antibodies that have been magnetically labeled, DAPI, or a combination thereof. In some embodiments, the methods disclosed herein further comprise contacting the population of CD146+ cells with anti-CD45 antibodies that have been fluorescently labeled, anti-CD105 antibodies that have been fluorescently labeled, anti-CD34 antibodies that have been fluorescently labeled, anti-CD31 antibodies that have been fluorescently labeled, anti-VEGFR2 antibodies that have been fluorescently labeled, anti CD-144 antibodies that have been fluorescently labeled, anti-CD106 antibodies that have been fluorescently labeled, DAPI, or a combination thereof.

In some embodiments, the methods disclosed herein further comprise discarding a CD146+ cell that specifically binds to an anti-CD45 antibody, to generate a population of isolated circulating endothelial cells. In some embodiments, the methods disclosed herein further comprise retaining a CD146+ cell that does not specifically bind to an anti-CD45 antibody, to generate a population of isolated circulating endothelial cells. In some embodiments, the methods disclosed herein further comprise discarding a CD 146+ cell that specifically binds to an anti-CD45 antibody and retaining a CD146+ cell that does not specifically bind to an anti-CD45 antibody, to generate a population of isolated circulating endothelial cells.

Other methods of isolating CECs based on physical properties of the CEC'for example size, charge, and density may be used. Size could be selected by filtration, density could be selected by Ficoll separation and charge could be selected by electrophoresis or dielectrophoresis. In some embodiments, the isolation methods disclosed herein eliminate or reduce the variability that may accompany the processing of large sample volumes. In some embodiments the isolation methods disclosed herein allow for better reproducibility of results by reducing the inter-operator variability.

### Treatment of Cardiovascular Disorders

Disclosed herein, in certain embodiments, are methods of analyzing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard. In some embodiments, the methods further comprise prescribing a treatment regimen to the individual based on the amount by which the morphological feature(s) of the population of circulating endothelial cells deviates from the control or standard.

Disclosed herein, in certain embodiments, are methods of diagnosing a cardiovascular disorder in an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; and (b) comparing a morphological feature of the population of circulating endothelial cells in the magnified image to a same morphological feature of a control or standard; and (c) diagnosing a cardiovascular disorder in the individual if the morphological feature(s) of the population of circulating endothelial cells (i) deviates from the control or standard from a healthy individual or population of healthy individuals, or (ii) matches the control or standard from an individual or population of individuals who have or have had the cardiovascular disease. In some embodiments, the methods further comprise prescribing a treatment regimen to the individual based on the diagnosis.

Disclosed herein, in certain embodiments, are methods of predicting whether an individual is susceptible to developing a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the susceptibility of the individual for developing a cardiovascular disorder based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. In some embodiments, the methods further comprise prescribing a treatment regimen to the individual based on the prediction.

Disclosed herein, in certain embodiments, are methods of predicting an individual's response to a treatment regimen for a cardiovascular disorder, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) predicting the individual's response to a treatment regimen based on (i) the deviation of the morphological feature(s) of the population of circulating endothelial cells from a control or standard derived from a healthy individual or population of healthy individuals, or (ii) the similarity of the morphological feature(s) of the population of circulating endothelial cells and a control or standard derived from an individual or population of individuals who have or have had the cardiovascular disease. In some embodiments, the methods further comprise prescribing a treatment regimen to the individual or altering a treatment regimen prescribed to the individual based on the amount by which the morphological feature(s) of the population of circulating endothelial cells deviates from the control or standard.

Disclosed herein, in certain embodiments, are methods of prescribing a treatment regimen for a cardiovascular disorder to an individual in need thereof, comprising: (a) obtaining a magnified image of a population of circulating endothelial cells (CECs) isolated from a blood sample obtained from the individual, wherein the blood sample comprises cells selected from circulating endothelial cells and circulating non-endothelial cells; (b) comparing a morphological feature of the population of circulating endothelial cells in the magnified image to a same morphological feature of a control or standard; and (c) prescribing a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease.

In some embodiments, the treatment regimen is drug therapy; enhanced external counterpulsation (EECP); surgery; or a combination thereof. In some embodiments, the treatment regimen is angioplasty; coronary artery bypass surgery; or a combination thereof

In some embodiments, the treatment regimen is drug therapy. In some embodiments, the treatment is a drug therapy selected from: niacin; a fibrate; a statin; an apolipoprotein A-1 modulator; an ACAT modulator; a CETP modulator; a glycoprotein IIb/IIIa modulator; a P2Y12 modulator; an Lp-PLA2 modulator; or combinations thereof In some embodiments, the treatment is a drug therapy selected from: atorvastatin; cerivastatin; fluvastatin; lovastatin; mevastatin; pitavastatin; pravastatin; rosuvastatin; simvastatin; simvastatin and ezetimibe; lovastatin and niacin, extended-release; atorvastatin and amlodipine besylate; simvastatin and niacin, extended-release; or combinations thereof. In some embodiments, the treatment is a drug therapy selected from: bezafibrate; ciprofibrate; clofibrate; gemfibrozil; fenofibrate; or combinations thereof. In some embodiments, the treatment is a drug therapy selected from: is DF4 (Ac-D-W-F-K-A-F-Y-D-K-V-A-E-K-F-K-E-A-F-NH2); DF5; RVX-208 (Resverlogix); or combinations thereof. In some embodiments, the treatment is a drug therapy selected from: avasimibe; pactimibe sulfate (CS-505); CI-1011 (2,6-diisopropylphenyl [(2, 4,6-triisopropylphenyl)acetyl]sulfamate); CI-976 (2,2-dimethyl-N-(2,4,6-trimethoxyphenyl)dodecanamide); VULM1457 (1-(2,6-diisopropyl-phenyl)-3-[4-(4'-nitrophenylthio)phenyl] urea); CI-976 (2,2-dimethyl-N-(2,4,6- trimethoxyphenyl)dodecanamide); E-5324 (n-butyl-N'-(2-(3-(5-ethyl-4-phenyl-1H-imidazol-1-yl)propoxy)-6-methylphenyl)urea); HL-004 (N-(2,6-diisopropylphenyl) tetradecylthioacetamide); KY-455 (N-(4,6- dimethyl-1-pentylindolin-7-yl)-2,2-dimethylpropanamide); FY-087 (N-[2-[N'-pentyl-(6,6-dimethyl-2,4-heptadiynyl)amino]ethyl]-(2-methyl-1-naphthyl-thio)acetamide); MCC-147 (Mitsubishi Pharma); F 12511 ((S)-2',3',5'-trimethyl-4'-hydroxy-alpha-dodecylthioacetanilide); SMP-500 (Sumitomo Pharmaceuticals); CL 277082 (2,4-difluoro-phenyl-N[[4-(2,2-dimethylpropyl)phenyl]methyl]-N-(hepthyl)urea); F-1394 ((1s,2s)-2-[3-(2,2-dimethylpropyl)-3-nonylureido]aminocyclohexane-1-yl 3-[N-(2,2,5,3-tetramethyl-],3-dioxane-4-carbonyl)amino]propionate); CP- 113818 (N-(2,4-bis(methylthio)-6-methylpyridin-3-yl)-2-(hexylthio)decanoic acid amide); YM-750; or combinations thereof In some embodiments, the treatment is a drug therapy selected from: torcetrapib; anacetrapid; JTT-705 (Japan Tobacco/Roche); or combinations thereof. In some embodiments, the treatment is a drug therapy selected from: abciximab; eptifibatide; tirofiban; roxifiban; variabilin; XV 459 (N(3)-(2-(3-(4-formamidinophenyl)isoxazolin-5-yl)acetyl)-N(2)-(1-butyloxycarbonyl)-2,3-diaminopropionate); SR 121566A (3-[N-{4-[4-(aminoiminomethyl)phenyl]-1,3-thiazol-2-yl}-N-(1 - carboxymethylpiperid-4-yl) aminol propionic acid, trihydrochloride); FK419 ((S)-2-acetylamino-3-[(R)-[1-[3-(piperidin-4-yl) propionyl] piperidin-3-ylcarbonyl] amino] propionic acid trihydrate); or combinations thereof. In some embodiments, the treatment is a drug therapy selected from: clopidogrel; prasugrel; cangrelor; AZD6140 (AstraZeneca); MRS 2395 (2,2-Dimethyl-propionic acid 3-(2-chloro-6-methylaminopurin-9-yl)- 2-(2,2-dimethylpropionyloxymethyl)-propyl ester); BX 667 (Berlex Biosciences); BX 048 (Berlex Biosciences) or combinations thereof. In some embodiments, the treatment is a drug therapy selected from: darapladib (SB 480848); SB-435495 (GlaxoSmithKline); SB-222657 (GlaxoSmithKline); SB-253514 (GlaxoSmithKline); or combinations thereof. In some embodiments, the treatment is a drug therapy selected from: A-81834 (3-(3-(1,1-dimethylethylthio-5-(quinoline-2-ylmethoxy)-1-(4-chloromethylphenyl)indole-2-yl)-2,2-dimethylpropionaldehyde oxime-O-2-acetic acid; AME103 (Amira); AME803 (Amira); atreleuton; BAY-x-1005 ((R)-(+)-alpha-cyclopentyl-4-(2-quinolinylmethoxy)-Benzeneacetic acid); CJ-13610 (4-(3-(4-(2-Methyl-imidazol-1-yl)-phenylsulfanyl)- phenyl)-tetrahydro-pyran-4-carboxylic acid amide); DG-031 (DeCode); DG-051 (DeCode); MK886 (1-[(4-chlorophenyl)methyl]3-[(1,1-dimethylethyl)thio]-α,α-dimethyl-5-(1-methylethyl)-1H-indole-2-propanoic acid, sodium salt); MK591 (3-(1-4[(4-chlorophenyl)methyl]-3-[(t-butylthio)-5-((2-quinoly)methoxy)-1H-indole-2]-, dimehtylpropanoic acid); RP64966 ([4-[5-(3-Phenyl-propyl)thiophen-2- yl]butoxy] acetic acid); SA6541 ((R)-S-[[4-(dimethylamino)phenyl]methyl] -N-(3-mercapto-2methyl- 1 -oxopropyl-L-cycteine); SC-56938 (ethyl-1-[2-[4-(phenylmethyl)phenoxy] ethyl] -4-piperidine- carboxylate); VIA-2291 (Via Pharmaceuticals); WY-47,288 (2-[(1-naphthalenyloxy)methyl]quinoline); zileuton; ZD-2138 (6-((3-fluoro-5- (tetrahydro-4-methoxy-2H-pyran-4yl)phenoxy)methyl)-1-methyl-2(1H)-quinlolinone); or combinations thereof

In some embodiments, the treatment regimen is EECP. EECP involves the placement of pneumatic cuffs on the legs. The cuffs are connected to telemetry monitors that monitor heart rate and rhythm and are timed to inflate and deflate based on the individual's electrocardiogram. The cuffs should inflate at the beginning of diastole and deflate at the beginning of systole. In some embodiments, the treatment regimen is angioplasty. Angioplasty involves the insertion of a balloon catheter into an artery. The catheter is positioned at the site of an atherosclerotic plaque and is inflated to a fixed size. The balloon crushes the plaque, opening up the blood vessel.

In some embodiments, the treatment regimen is coronary artery bypass surgery. Coronary artery bypass surgery is a surgical procedure wherein arteries or veins from the patient (or, a donor) are grafted to a coronary artery with atherosclerotic narrowing.

### Examples

### Materials and Methods for Examples 1 and 2

### Patient selection and specimen collection

Between January 2010 and February 2011, patients presenting to the emergency room with STEMI at 4 regional medical centers in San Diego County had blood drawn for CEC characterization. All STEMI blood samples were obtained in the cardiac catheterization laboratory via an arterial sheath and prior to catheter insertion for diagnostic coronary angiography or intervention. All patients met criteria for STEMI including ST-segment elevation of at least 0.2 mV in 2 contiguous precordial leads or 0.1 mV in contiguous limb leads. Positive markers of myocardial ischemia (CK-MB or troponin) and angiographic evidence of obstructive coronary artery disease were also required. Initial cardiac troponin and CK-MB values were obtained and recorded within 90 minutes of presentation. Subsequent CK-MB and troponin values were not recorded.

Healthy controls were recruited from the normal blood donor program at The Scripps, Research Institute for the purpose of comparing CEC levels and morphology to that obtained from STEMI patients. All healthy controls were between the ages of 18 and 35 and were deemed free of any chronic disorders via self-report. Blood for CEC ascertainment in healthy and random age-matched samples were obtained via venipuncture. Randomly selected samples of age-matched controls that via self-report denied the presence of any acute illnesses or symptoms were included in the CEC morphology analysis portion of the study. Smoking status, obesity, family history of cardiovascular disease, or acute febrile illness was not used as exclusionary criteria. Individuals with existing vascular disease who recently underwent an open endarterectomy procedure along with patients with non ST-segment elevation myocardial infarction (NSTEMI) were also recruited for the sole purpose of morphologic characterization of their CECs. Criteria for MI in the NSTEMI population included symptoms consistent with MI, serologic evidence of myocardial necrosis - as determined by elevated levels of cardiac troponins and CK-MB, and angiographic evidence of obstructive coronary artery disease.

All blood from cases and controls were collected in tubes containing a mild cellular fixative known to stabilize CEC levels for up to 72 hours. Subsequently, samples were kept at room temperature and shipped via courier to a central lab for processing within 48 hours of collection. Institutional review board approval was obtained from all recruiting sites, and all patients gave informed consent.

### Identification of CECs

Briefly, CECs in whole blood were bound by anti-CD146 antibody conjugated magnetic nanoparticles, and enriched by repeated magnetic incubations and automated washings. CD146+ enriched cells were stained with fluorescent antibodies to CD105 and CD45, and the magnetically enriched and fluorescent antibody labeled cells placed into a MagNest® Cell Presentation Device. Figure 1A shows the steps during sample and image analysis by the CellTracks Analyzer II (CTAII). The MagNest® device consists of a disposable sample cartridge positioned between two permanent magnets in order to orient the magnetically labeled cells in a monolayer for fluorescent image analysis. The MagNest® is placed in the CTA II, a four-color semi-automated fluorescent microscope. The analyzer then scans the entire cartridge surface collecting images for each of the four fluorescent colors. It records 180 images for each fluorescent channel (720 images per scan). The CTA II's software automatically analyzes each frame and identifies those objects within the frame that based on their DAPI and CD105 fluorescence were possible candidate CECs. Candidate CECs are placed as a series of thumbnails in an image gallery for review and identification by a trained operator (Supplementary Fig 2 A). The thumbnail images show, from right to left, an unused (FITC) channel, CD45-APC signal, DAPI stained nuclei, CD105-PE reactivity, and finally an overlay of the CD105-PE & DAPI staining. The FITC channel can be used to phenotype CECs with additional markers of interest. To be scored as a CEC a cell had to have a nucleus, express CD105, have the morphology of a cell, and be negative for CD45. Supplementary Fig 2B shows an example of 3 objects as presented to the reviewer by the CTA II software. The first two objects met the criteria and were scored as CECs by the operator (checked box). The third object was judged a leukocyte because it was CD45 positive (box not checked). The software module automatically tabulated the checked boxes within each sample, and results were expressed as the number of CECs per 4 mL ofblood.

### CEC morphology analysis

CEC image analysis was performed using a customized Matlab program. Data analysis was performed either using Matlab or GraphPad Prism (release 4.00). Fluorescence intensity measurements of fixed CECs from CellSearch (Veridex) were carried out in a four-step process: (1) Cropping and separation of single CEC color images into their individual channels (CD105 and DAPI), which were then saved as tifs. (2) Cell body (CD105) and nuclear (DAPI) segmentation. (3) Area calculation of the segmented images. (4) Single cell and nuclear area calculation. Two Gaussian transformations with s.d. σ1 and σ2 were calculated for the CD105 and DAPI channels of each CEC image. The background pixels were removed using unimodal thresholding28 and the final objects corresponding to CEC cell body and nuclei were determined as thresholded difference of two Gaussians images. The set of s.d. σ1 and σ2 used in the Gaussian transformations was chosen to generate the most accurate mask by visual inspection. The area of the CD105 channel for each CEC image was divided by the number of nuclei identified in the nuclear masks contained within the CD 105 area. Data analysis was performed either using Matlab or GraphPad Prism. All statistical comparisons were performed using pairwise t-test (two tails) in GraphPad Prism.

### Statistical analysis of CEC counts

A two-sample test for the nonparametric Behrens-Fisher problem was used to see whether STEMI cases exhibited higher counts of CECs compared to controls. Spearman rank correlation was used to determine the linear relationship between the number of CECs and levels of MB, troponin, as well as age. A mathematical model was built using logistic regression with ten-fold cross-validation that classified patients into two groups, STEMI and controls, based on their observed CEC counts. This model generated in Weka software was used to assess the number of correctly classified instances and the area under the receiver-operating characteristic (ROC) curve. The ROC curve shown in Fig. 2B is based on a logistic regression model using all available data (i.e., without cross-validation), and was generated using the ROCR package of the R statistical computing environment. Area under the ROC curve (AUC) was calculated using somers2 function of the Hmisc package in the R statistical computing environment.

### Example 1: CEC levels are diagnostic of arterial injury in acute myocardial infarction.

CECs were unambiguously identified based on three criteria: positive staining for nuclei, positive staining for CD-105 (an EC marker), and negative staining for CD-45 (a leukocyte marker)(Fig. 1).

Fifty consecutive STEMI (82% male) patients were prospectively enrolled for CEC level ascertainment. The median CEC count in STEMI patients was 19 CECs/ml with upper and lower quartiles of 12 CECs/ml and 51 CECs/ml (range 2.5 - 465 CECs/ml) respectively (Table 1, Fig. 2).

**Table 1**

| | Number | Age | % Male | CECs/mL | Troponin | CK-MB |
|---|---|---|---|---|---|---|
| STEMI | 50 | 58.5 (39-80) | 82% | 19.4 (11.6, 51.1) | 5.2 (1.7, 17.8) | 26.9 (8.4, 81.9) |
| Controls | 44 | 30 (22-34) | 51% | 3.8 (2.3, 5.1) | --- | --- |

The median age was 58.5 years (range 39-80 years) with the median cardiac troponin and CK-MB values being 5.7 ng/ml and 27.9 ng/ml respectively. Quartiles and ranges for cardiac troponins and CK-MB values in all STEMI cases are shown in Table 1. Notably, the STEMI cases had CK-MB and troponin levels that were on average logarithmically higher than clinically accepted normal values (troponin < 0.1 ng/ml; CK-MB <3.0 ng/ml) and were well within the range expected for STEMI cases. Importantly, these values underscore the significant myocardial necrosis that typically accompanies this phenotype. No correlation was observed between CEC counts and initial intramyocardial enzyme levels (p=0.02, CK-MB; p =-0.03, troponin) (Fig. 3).

Blood samples for CEC enumeration were also prospectively obtained from 44 consecutive healthy controls. The median age of the healthy controls was 30 years (range 22 - 34). The median number of CECs/mL was 3.8 CECs/ml (range 0.75 -16.75) with upper and lower quartile thresholds being 2.25 CECs/ml and 5.06 CECs/ml respectively (Table 1, **Fig**. **2**). Importantly, the first 24 healthy controls enrolled were brought back for a repeat measurement of CEC numbers at two months (**Fig**. **4**). Notably, the median number of CECs/ml were unchanged between the first and second visits with median values being 3.4 CECs/ml and 3.25 CECs/ml during the first and second visits respectively (P=0.5). These data suggest that CEC levels tend to remain stable over time in healthy individuals. Further, there was no correlation between CEC counts and age in the healthy controls and the random age-matched population sample that was included for the CEC morphology portion of the study (p =-0.12)(**Fig**. **5**).

We observed CEC counts to be dramatically elevated in our STEMI population when compared to healthy controls (**Fig**. **2**, P=1.1x10-10). A receiver-operating characteristic (ROC) curve associated with a classifier based upon logistic regression demonstrated an area under the ROC curve of 0.95 (AUC of 1 would suggest perfect classification, AUC equal to 0.5 would indicate random selection) (**Fig**. **2B**). This classifier was able to correctly classify 86 (91.5%) of all instances (i.e. CEC/ml counts for 50 patients with STEMI and 44 healthy controls) as a MI case or healthy control with ten-fold cross-validation. For illustration, a value of 16.5 CECs/ml had a sensitivity of 60%, but an impressive 98% specificity in the diagnosis of STEMI (**Fig**. **2B**). Alternatively, at a classification threshold of 9 CECs/ml, the associated sensitivity is equal to 90%, while the specificity reduced slightly to 93%. In summary, CEC counts appear to be independent and predictive biomarkers of arterial injury in individuals with acute myocardial infarction.

### Example 2: CECs from MI patients are abnormally large and misshapen and often appear with multiple nuclei

A detailed analysis of fluorescent images of isolated CECs from 8 STEMI cases and 10 healthy controls was performed. STEMI cases and healthy controls with CEC numbers in the low, middle, and high range were selected for analysis (**Table 2**).

**Table 2**

| **Category** | **MI cases/ controls Identification number** | **Age (years)** | **CEC/1ml Blood** |
|---|---|---|---|
| **MI cases** | | | |
| STEMI | CEC03067 | 39 | 4.5 |
| STEMI | CEC2A040 | 70 | 11.5 |
| STEMI | CEC03040 | 53 | 58.5 |
| STEMI | CEC1D004 | 58 | 114 |
| STEMI | CEC03063 | 54 | 16.75 |
| STEMI | CEC2B003 | 56 | 465.25 |
| STEMI | CEC03048 | 56 | 107.25 |
| STEMI | CEC03062 | 39 | 12 |
| **MI cases** | | | |
| NSTEMI | CEC03035 | 57 | 99 |
| NSTEMI | CEC2A027 | 71 | 109.75 |
| **Healthy controls** | CEC1B006 | 31 | 2.5 |
| | CEC1B013 | 33 | 1.25 |
| | CEC1B017 | 29 | 1.25 |
| | CEC1B001 | 32 | 5 |
| | CEC1B003 | 33 | 12 |
| | CEC1B014 | 27 | 2.5 |
| | CEC1B009 | 31 | 9.75 |
| | CEC1B044 | 26 | 3.5 |
| | CEC1B023 | 28 | 8.75 |
| | CEC1B026 | 34 | 1.25 |
| **Age-matched controls** | Pt 219 | 63 | 4.75 |
| | Pt 223 | 57 | 3.75 |
| | Pt 259 | 51 | 4.25 |
| | Pt 265 | 62 | 2.25 |
| | Pt 206 | 51 | 8.25 |
| | Pt 266 | 50 | 1 |
| | Pt 267 | 62 | 2.25 |
| | Pt 268 | 54 | 10.25 |
| | Pt 274 | 63 | 6.25 |
| | Pt 275 | 63 | 1 |
| **Vascular controls** | CEC1A012 | 74 | 1.75 |
| | CEC1A013 | 67 | 1 |

Additional control populations were also included for this portion of the study, so that any conclusions derived from the morphologic appearance of STEMI CECs could be placed in the context of real-world clinical populations. Specifically, 10 consecutive population controls that were age-matched to our STEMI patient group were selected. Additionally, 2 patients with documented peripheral arterial disease who had recently undergone an open vascular procedure were included as a vascular disease control group. Two patients with Non ST-segment elevation myocardial infarction (NSTEMI) were also included in order to assess any potential differences in CEC morphology that might be distinguished between the two MI phenotypes (STEMI vs. NSTEMI).

Visual inspection of individual fluorescent CEC images revealed that morphologies of CECs isolated from MI patients are strikingly different than those of CECs from controls. **Figure 6** illustrates individual CECs isolated from healthy and age-matched controls, compared to CECs isolated from a representative STEMI patient (CEC03040). CECs from controls are relatively small, tend to be elongated in shape, and occasionally have two nuclei associated with one cell body, which may represent a cluster of two attached cells, or one cell with two nuclei (**Fig**. **6A**). In contrast, CECs from the STEMI patient are more heterogeneous in appearance, with many cells that are obviously larger and grossly misshapen, while others are smaller (**Fig**. **6B**). The nuclei of CECs from the STEMI patient also tend to be larger and aberrantly shaped as compared to nuclei of CECs from control individuals (**Fig**. **6B**, also see **Fig**. **7D**). Many of the largest CEC images from this STEMI patient often contain several nuclei associated with one continuous CD105-stained cell body region, thus appearing as multi-nuclear clusters with up to 6 nuclei (**Fig**. **6B**) (also see below, **Fig**. **8**). Since cell-cell boundaries are not clearly demarcated in these fluorescence images, it is not possible to determine rigorously whether these are multi-cellular clusters, or alternatively, multi-nuclear individual cells.

Cellular and nuclear areas for individual images of CECs (**Fig**. **5**) were determined. CEC morphologies in 50-100 randomly selected images were analyzed for each MI patient, and compared to all CEC images from control patients. This revealed that the mean cellular area of CECs from MI patients was ~270 mm2, more than 2.5-fold greater than the mean ~100 mm2 cellular area of CECs from either healthy or age-matched controls (P<0.0001) (**Fig**. **7A**). Similarly, the mean area of CEC nuclei from MI patients was ~65 mm2, significantly larger than the 30 to 40 mm2 area for CEC nuclei from healthy and age-matched controls (P<0.0001) (**Fig**. **7B**). In contrast, the morphological parameters for CECs from patients undergoing vascular surgery for non-cardiac related conditions were not significantly different from controls (**Fig**. **7A and B**). Finally, the ratio of cellular/nuclear area was ~1.5 fold greater for CECs from MI patients as compared to CECs from any of the control groups, with a significance level at P<0.0001. Representative examples of the ranges of cellular and nuclear size variations for CEC images of single cells from the control and MI patients analysed are shown in **Fig. 7D****.**

While the cellular and nuclear areas of CECs from age-matched controls tended to be larger than those of CECs from healthy, younger controls (**Fig**. **7A and B**), the cellular/nuclear area ratios were not different between these two control groups (**Fig**. **7D**). Thus, the cellular and nuclear areas increased proportionately for CECs from age-matched compared to healthy controls, whereas the cellular area increased disproportionately to the nuclear area for CECs from MI patients. Therefore, the increased cellular/nuclear area ratio is a morphological abnormality specific to MI patients, since it is not observed for either age-matched or vascular control patients in comparison to healthy controls. A comparison between the STEMI and NSTEMI CECs that constitute the MI group analyzed for the cellular and nuclear areas as well as the cellular/nuclear area ratios, did not show any significant difference between the two groups (**Fig**. **9**).

The percent of CECs present as single cells with one nucleus, or as cells with 2 or more nuclei, for CECs from MI patients and controls, was determined. Examples of individual CEC images with multiple nuclei (from MI patients) are illustrated in **Figure 8A****.** Quantitative analysis revealed that on average, ~25% of the CEC images from MI patients contained 2 or more nuclei, compared to 5-10% of CEC images from the healthy or age-matched controls, which is a several-fold increase in the numbers of nuclei per image (P = 0.0008) (**Fig**. **9B**). While the percent of CEC images with multiple nuclei also appeared to be increased in the age-matched control group with respect to the healthy control or vascular group, this difference was not statistically significant due to the variability in this parameter in these two groups (P = 0.22) (**Fig**. **9B**). The distribution of CEC images containing from 2 up to 10 nuclei demonstrates that MI patients are the only group to contain more than 3 nuclei/image (**Fig**. **9C**). In contrast, the vast majority of CEC images from healthy, age-matched or vascular patients contained only 1 or 2 nuclei, with the exception of two and four CEC images with 3 nuclei from the healthy and the age-matched control groups, respectively (**Fig**. **9C**). 10-15% of CEC images in both control and MI patients contain 2 nuclei, which may represent individual bi-nuclear cells (a characteristic feature of mature endothelium). However, inspection of individual CEC images from MI patients with multiple (> 2) nuclei reveals a multi-lobular morphology in many cases, suggesting that at least some of these images are due to groups of several CECs attached to one another. Previous studies have also suggested that images of CECs from MI patients showing multiple nuclei, could constitute multi-cellular clusters.

### Example 3: Analysis of Individual during Routine Physical

An individual, 35 years of age, is undergoing a routine physical. The individual shows no signs of a cardiovascular disorder. A blood sample is obtained. The concentration of CECs in the blood of the individual is 200% higher than the standard concentration. The CECs are examined. The average cellular area is 40% larger than the standard cellular area. The average nuclear area is 40% larger than the standard nuclear area. Further, a large number of the CECs are abnormally shaped. Finally, 45% of the CECs are polynucleated. The individual is flagged as having a cardiovascular disorder. Further tests are performed and the individual is diagnosed with atherosclerosis.

### Example 4: Analysis of Individual Presenting with Unstable Angina

An individual, 65 years of age, presents with chest pains. The individual indicates that his chest pains began 5 weeks before and that they occur when he is resting or sleeping. The individual further indicates that the pains tend to last for 30 minutes or more before they dissipate. A blood sample is obtained. The concentration of CECs in the blood of the individual is350% higher than the standard concentration. The CECs are examined. The average cellular area is 50% larger than the standard cellular area. The average nuclear area is 30% larger than the standard nuclear area. Further, a large number of the CECs are abnormally shaped. Finally, 40% of the CECs are polynucleated. The individual is flagged as having a cardiovascular disorder. Further tests are performed and the individual is diagnosed with atherosclerosis and at high risk for a myocardial infarction. The individual is taken to surgery and angioplasty is performed.

### Example 5: Analysis of Individual Presenting with Angina

An individual, 40 years of age, presents with chest pains. The individual indicates that his chest pains began 1 month before and that they occur when he is exercising. The individual further indicates that the pains tend to dissipate when exercise ceases. A blood sample is obtained. The concentration of CECs in the blood of the individual is 200% higher than the standard concentration. The CECs are examined. The average cellular area is 30% larger than the standard cellular area. The average nuclear area is 20% larger than the standard nuclear area. Further, a large number of the CECs are abnormally shaped. Finally, 10% of the CECs are polynucleated. The individual is flagged as having a cardiovascular disorder. Further tests are performed and the individual is diagnosed with mild atherosclerosis. Based on the CEC analysis the individual is predicted to respond well to administration of statins and a low fat diet. Statins are prescribed. The individual returns for a follow-up after 1 month and the atherosclerotic plaque is decreasing.

## Claims

1. A method of analyzing a cardiovascular disorder in an individual in need thereof, comprising:
(a) obtaining a magnified image of a population of circulating endothelial cells (CECs) isolated from a blood sample obtained from the individual; and
(b) comparing two or more morphological features of the population of circulating endothelial cells in the magnified image to a same morphological feature of a control or standard, wherein the morphological feature is: cellular area, nuclear area, ratio of cellular area to nuclear area, cell shape, nuclei shape, number of nuclei, number of circulating endothelial cells, or a combination thereof.

2. The method of claim 1, further comprising diagnosing the individual with a cardiovascular disorder if the morphological feature(s) of the population of circulating endothelial cells (a) deviates from the control or standard from a healthy individual or population of healthy individuals, or (b) matches the control or standard from an individual or population of individuals who have or have had the cardiovascular disease.

3. The method of claim 1, further comprising predicting the susceptibility of the individual for developing a cardiovascular disorder based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease.

4. The method of claim 1, further comprising prescribing a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease.

5. The method of claim 1, further comprising predicting the individual's response to a treatment regimen based on (a) the deviation of the morphological feature(s) of the population of circulating endothelial cells from a control or standard derived from a healthy individual or population of healthy individuals, or (b) the similarity of the morphological feature(s) of the population of circulating endothelial cells and a control or standard derived from an individual or population of individuals who have or have had the cardiovascular disease.

6. The method of any of claims 2 to5, wherein
the deviation is the population of circulating endothelial cells having an average cellular area that is greater than the cellular area of CECs from a control or standard derived from a healthy individual or population of healthy individuals;
the deviation is the population of circulating endothelial cells having an average nuclear area that is greater than the nuclear area of CECs from a control or standard derived from a healthy individual or population of healthy individuals;
the deviation is the population of circulating endothelial cells having an average ratio of cellular area to nuclear area that is greater than the average ratio of cellular area to nuclear area of CECs from a control or standard derived from a healthy individual or population of healthy individuals;
the deviation is the population of circulating endothelial cells having an average number of nuclei that is greater than the number of nuclei of CECs from a control or standard derived from a healthy individual or population of healthy individuals;
the deviation is the population of circulating endothelial cells having an abnormal cellular shape as compared to the cellular shape of CECs from a control or standard derived from a healthy individual or population of healthy individuals; or
the deviation is the population of circulating endothelial cells having an abnormal nuclear shape as compared to the nuclear shape of CECs from a control or standard derived from a healthy individual or population of healthy individuals.

7. The method of any of claims 2 to 5, wherein the deviation is the population of circulating endothelial cells having an average cellular area that is at least about 30% greater than the average cellular area of CECs from a control or standard derived from a healthy individual or population of healthy individuals.

8. The method of any of claims 2 to 5, wherein the deviation is the population of circulating endothelial cells having an average cellular area that is greater than about 100 µm².

9. The method of any of claims 2 to 5, wherein the deviation is the population of circulating endothelial cells having an average nuclear area that is at least about 30% greater than the average nuclear area of CECs from a control or standard derived from a healthy individual or population of healthy individuals.

10. The method of any of claims 2 to 5, wherein the deviation is the population of circulating endothelial cells having an average nuclear area that is greater than about 40 µm².

11. The method of any of claims 2 to 5, wherein the deviation is the population of circulating endothelial cells having an average of at least two nuclei per CEC.

12. The method of any of claims 1 to11, further comprising comparing:
(a) the concentration of the population of circulating endothelial cells isolated from the blood sample; and
(b) the concentration of a control or standard.

13. The method of any of claims 1 to12, wherein the population of circulating endothelial cells is isolated from the blood sample by:
(a) contacting the blood sample with anti-CD146 antibodies, wherein the antibodies are magnetically labeled or fluorescently labeled; and
(b) isolating the cells to which the anti-CD146 antibodies specifically bind, to generate a population of CD 146+ cells.

14. The method of claim 13, further comprising contacting the population of CD 146+ cells with fluorescently labeled anti-CD45 antibodies, anti-CD105 antibodies, anti CD34 antibodies, anti-CD31 antibodies, anti-VEGF2 antibodies, anti-CD 144 antibodies, anti-CD 106 antibodies, DAPI, or a combination thereof.

15. The method of claim 13 or 14, further comprising discarding a CD146+ cell that specifically binds to an anti-CD45 antibody and retaining a CD 146+ cell that does not specifically bind to an anti-CD45 antibody, to generate a population of isolated circulating endothelial cells.

16. The method of any of claims 1 to 15, wherein the cardiovascular disorder is selected from: plaque rupture, plaque erosion, ischemia of the heart, reperfusion injury to the heart, atherosclerosis, acute coronary syndrome, ST-segment elevation myocardial infarction (STEMI), non-ST-segment elevation myocardial infarction (NSTEMI), unstable angina, ischemic stroke or a combination thereof.

## Patentansprüche

1. Verfahren zur Analyse einer Herz-Kreislauf-Störung in einem Individuum, das eine solche Analyse benötigt, wobei das Verfahren folgendes umfasst:
(a) Erhalten eines vergrößerten Bilds einer Population zirkulierender Endothelzellen (CECs), isoliert von einer von dem Individuum entnommenen Blutprobe; und
(b) Vergleichen von zwei oder mehr morphologischen Merkmalen der Population zirkulierender Endothelzellen in dem vergrößerten Bild mit einem gleichen morphologischen Merkmal einer Kontrolle oder eines Standards, wobei das morphologische Merkmal folgendes ist: eine zellulare Fläche, eine Kernfläche, ein Verhältnis der zellularen Fläche zur Kernfläche, eine Zellform, eine Kernform, eine Kernanzahl, eine Anzahl zirkulierender Endothelzellen oder eine Kombination dieser.

2. Verfahren nach Anspruch 1, ferner umfassend die Diagnose des Individuums mit einer Herz-Kreislauf-Störung, wenn das/die morphologische(n) Merkmal(e) der Population zirkulierender Endothelzellen (a) von der Kontrolle oder dem Standard eines gesunden Individuums oder der Population gesunder Individuen abweicht; oder (b) mit der Kontrolle oder dem Standard eines Individuums oder einer Population von Individuen übereinstimmt, welche die Herz-Kreislauf-Störung aufweisen oder aufgewiesen haben.

3. Verfahren nach Anspruch 1, ferner umfassend das Prognostizieren der Anfälligkeit des Individuums für die Entwicklung einer Herz-Kreislauf-Störung auf der Basis (a) der Abweichung des/der morphologischen Merkmals/Merkmale der Population zirkulierender Endothelzellen von einer Kontrolle oder einem Standard, die bzw. der von einem gesunden Individuum oder einer Population gesunder Individuen gewonnen worden ist; oder (b) der Übereinstimmung des/der morphologischen Merkmals/Merkmale der Population zirkulierender Endothelzellen und einer Kontrolle oder einem Standard, die bzw. der von einem Individuum oder einer Population von Individuen gewonnen worden ist, welche die Herz-Kreislauf-Störung aufweisen oder aufgewiesen haben.

4. Verfahren nach Anspruch 1, ferner umfassend das Verordnen einer Behandlungsweise auf der Basis (a) der Abweichung des/der morphologischen Merkmals/Merkmale der Population zirkulierender Endothelzellen von einer Kontrolle oder einem Standard, die bzw. der von einem gesunden Individuum oder einer Population gesunder Individuen gewonnen worden ist; oder (b) der Übereinstimmung des/der morphologischen Merkmals/Merkmale der Population zirkulierender Endothelzellen und einer Kontrolle oder einem Standard, die bzw. der von einem Individuum oder einer Population von Individuen gewonnen worden ist, welche die Herz-Kreislauf-Störung aufweisen oder aufgewiesen haben.

5. Verfahren nach Anspruch 1, ferner umfassend das Prognostizieren der Reaktion des Individuums auf eine Behandlungsweise auf der Basis (a) der Abweichung des/der morphologischen Merkmals/Merkmale der Population zirkulierender Endothelzellen von einer Kontrolle oder einem Standard, die bzw. der von einem gesunden Individuum oder einer Population gesunder Individuen gewonnen worden ist; oder (b) der Übereinstimmung des/der morphologischen Merkmals/Merkmale der Population zirkulierender Endothelzellen und einer Kontrolle oder einem Standard, die bzw. der von einem Individuum oder einer Population von Individuen gewonnen worden ist, welche die Herz-Kreislauf-Störung aufweisen oder aufgewiesen haben.

6. Verfahren nach einem der Ansprüche 2 bis 5, wobei:
die Abweichung die Population zirkulierender Endothelzellen mit einer durchschnittlichen Zellfläche ist, die größer ist als die Zellfläche der CECs einer Kontrolle oder eines Standards, die bzw. der von einem gesunden Individuum oder einer Population gesunder Individuen gewonnen worden ist;
die Abweichung die Population zirkulierender Endothelzellen mit einer durchschnittlichen Kernfläche ist, die größer ist als die Kernfläche der CECs einer Kontrolle oder eines Standards, die bzw. der von einem gesunden Individuum oder einer Population gesunder Individuen gewonnen worden ist;
die Abweichung die Population zirkulierender Endothelzellen mit einem durchschnittlichen Verhältnis der Zellfläche zu der Kernfläche ist, die größer ist als das durchschnittliche Verhältnis der Zellfläche zu der Kernfläche der CECs einer Kontrolle oder eines Standards, die bzw. der von einem gesunden Individuum oder einer Population gesunder Individuen gewonnen worden ist;
die Abweichung die Population zirkulierender Endothelzellen mit einer durchschnittlichen Kernanzahl ist, die größer ist als die Kernanzahl der CECs einer Kontrolle oder eines Standards, die bzw. der von einem gesunden Individuum oder einer Population gesunder Individuen gewonnen worden ist;
die Abweichung die Population zirkulierender Endothelzellen mit einer anomalen Zellform im Vergleich zu der Zellform der CECs einer Kontrolle oder eines Standards ist, die bzw. der von einem gesunden Individuum oder einer Population gesunder Individuen gewonnen worden ist; oder
die Abweichung die Population zirkulierender Endothelzellen mit einer anomalen Kernform im Vergleich zu der Kernform der CECs einer Kontrolle oder eines Standards ist, die bzw. der von einem gesunden Individuum oder einer Population gesunder Individuen gewonnen worden ist.

7. Verfahren nach einem der Ansprüche 2 bis 5, wobei die Abweichung die Population zirkulierender Endothelzellen mit einer durchschnittlichen Zellfläche ist, die um etwa 30% größer ist als die durchschnittliche Zellfläche der CECs einer Kontrolle oder eines Standards ist, die bzw. der von einem gesunden Individuum oder einer Population gesunder Individuen gewonnen worden ist.

8. Verfahren nach einem der Ansprüche 2 bis 5, wobei die Abweichung die Population zirkulierender Endothelzellen mit einer durchschnittlichen Zellfläche ist, die größer ist als etwa 100 µm².

9. Verfahren nach einem der Ansprüche 2 bis 5, wobei die Abweichung die Population zirkulierender Endothelzellen mit einer durchschnittlichen Kernfläche ist, die wenigstens etwa 30% größer ist als die durchschnittliche Kernfläche der CECs einer Kontrolle oder eines Standards ist, die bzw. der von einem gesunden Individuum oder einer Population gesunder Individuen gewonnen worden ist.

10. Verfahren nach einem der Ansprüche 2 bis 5, wobei die Abweichung die Population zirkulierender Endothelzellen mit einer durchschnittlichen Kernfläche ist, die größer ist als etwa 40 µm².

11. Verfahren nach einem der Ansprüche 2 bis 5, wobei die Abweichung die Population zirkulierender Endothelzellen mit durchschnittlich wenigstens zwei Kernen je CEC ist.

12. Verfahren nach einem der Ansprüche 1 bis 11, wobei dieses ferner das Vergleichen umfasst von:
(a) der Konzentration der Population von der Blutprobe isolierter zirkulierende Endothelzellen; und
(b) der Konzentration einer Kontrolle oder eines Standards.

13. Verfahren nach einem der Ansprüche 1 bis 12, wobei die Population zirkulierender Endothelzellen von der Blutprobe isoliert wird durch:
(a) Inkontaktbringen der Blutprobe mit Anti-CD146 Antikörpern, wobei die Antikörper magnetisch markiert oder fluoreszierend markiert sind; und
(b) Isolierend der Zellen, an welche die Anti-CD146 Antikörper spezifisch finden, um eine Population von CD146+ Zellen zu erzeugen.

14. Verfahren nach Anspruch 13, ferner umfassend das Inkontaktbringen von CD146+ Zellen mit fluoreszierend markierten Anti-CD45 Antikörpern, Anti-CD105 Antikörpern, Anti-CD34 Antikörpern, Anti-CD31 Antikörpern, Anti-VEGF2 Antikörpern, Anti-CD144 Antikörpern, Anti-CD106 Antikörpern, DAPI oder einer Kombination dieser.

15. Verfahren nach Anspruch 13 oder 14, ferner umfassend das Verwerfen einer CD146+ Zelle, die spezifisch an einen Anti-CD45 Antikörper bindet und Behalten einer CD146+ Zelle, die nicht spezifisch an einen Anti-CD45 Antikörper bindet, um eine Population isolierter zirkulierender Endothelzellen zu erzeugen.

16. Verfahren nach einem der Ansprüche 1 bis 15, wobei die Herz-Kreislauf-Störung ausgewählt ist aus: Plaqueruptur, Plaqueerosion, Ischämie des Herzens, Reperfusionsschaden des Herzens, Atherosklerose, akutem Koronarsyndrom, Myokardinfarkt mit ST-Streckenhebung (STEMI), Nicht-ST-Hebungsinfarkt (NSTEMI), instabiler Angina, ischämischem Schlaganfall oder einer Kombination dieser.

## Revendications

1. Méthode d'analyse d'un trouble cardiovasculaire chez un individu ayant besoin d'une telle analyse, comportant les étapes consistant à :
(a) obtenir une image agrandie d'une population de cellules endothéliales circulantes (CEC) isolées d'un échantillon de sang obtenu de l'individu ; et
(b) comparer deux caractéristiques morphologiques ou plus de la population de cellules endothéliales circulantes dans l'image agrandie à une même caractéristique morphologique d'un contrôle ou d'une référence, la caractéristique morphologique étant : surface cellulaire, surface nucléaire, rapport de la surface cellulaire sur la surface nucléaire, forme de la cellule, forme du noyau, nombre de noyaux, nombre de cellules endothéliales circulantes, ou une combinaison de celles-ci.

2. Méthode selon la revendication 1, comprenant en outre l'étape consistant à diagnostiquer l'individu souffrant d'un trouble cardiovasculaire si la(les) caractéristique(s) morphologique(s) de la population de cellules endothéliales circulantes (a) dévie(nt) du contrôle ou de la référence d'un individu sain ou d'une population d'individus sains, ou (b) correspond(ent) au contrôle ou à la référence d'un individu ou d'une population d'individus qui souffrent ou ont souffert du trouble cardiovasculaire.

3. Méthode selon la revendication 1, comportant en outre les étapes consistant à prévoir la susceptibilité de l'individu à développer un trouble cardiovasculaire en fonction de (a) la déviation de la(des) caractéristique(s) morphologique(s) de la population de cellules endothéliales circulantes d'un contrôle ou d'une référence provenant d'un individu sain ou d'une population d'individus sains, ou (b) la similitude de la(des) caractéristique(s) morphologique(s) de la population de cellules endothéliales circulantes et d'un contrôle ou d'une référence provenant d'un individu ou d'une population d'individus qui souffrent ou ont souffert du trouble cardiovasculaire.

4. Méthode selon la revendication 1, comportant en outre l'étape consistant à prescrire un régime de traitement en fonction (a) la déviation de la(des) caractéristique(s) morphologique(s) de la population de cellules endothéliales circulantes d'un contrôle ou d'une référence provenant d'un individu sain ou d'une population d'individus sains, ou (b) la similitude de la(des) caractéristique(s) morphologique(s) de la population de cellules endothéliales circulantes et d'un contrôle ou d'une référence provenant d'un individu ou d'une population d'individus qui souffrent ou ont souffert du trouble cardiovasculaire.

5. Méthode selon la revendication 1, comportant en outre l'étape consistant à prévoir la réaction de l'individu à un régime de traitement en fonction de (a) la déviation de la(des) caractéristique(s) morphologique(s) de la population de cellules endothéliales circulantes d'un contrôle ou d'une référence provenant d'un individu sain ou d'une population d'individus sains, ou (b) la similitude de la(des) caractéristique(s) morphologique(s) de la population de cellules endothéliales circulantes et d'un contrôle ou d'une référence provenant d'un individu ou d'une population d'individus qui souffrent ou ont souffert du trouble cardiovasculaire.

6. Méthode selon l'une quelconque des revendications 2 à 5,
la déviation étant la population de cellules endothéliales circulantes ayant une surface cellulaire moyenne qui est supérieure à la surface cellulaire des CEC d'un contrôle ou d'une référence provenant d'un individu sain ou d'une population d'individus sains ;
la déviation étant la population de cellules endothéliales circulantes ayant une surface nucléaire moyenne qui est supérieure à la surface nucléaire des CEC d'un contrôle ou d'une référence provenant d'un individu sain ou d'une population d'individus sains ;
la déviation étant la population de cellules endothéliales circulantes ayant un rapport moyen de la surface cellulaire sur la surface nucléaire qui est supérieur au rapport moyen de la surface cellulaire sur la surface nucléaire des CEC d'un contrôle ou d'une référence provenant d'un individu sain ou d'une population d'individus sains ;
la déviation étant la population de cellules endothéliales circulantes ayant un nombre moyen de noyaux qui est supérieur au nombre de noyaux des CEC d'un contrôle ou d'une référence provenant d'un individu sain ou d'une population d'individus sains ;
la déviation étant la population de cellules endothéliales circulantes ayant une forme cellulaire anormale par rapport à la forme cellulaire des CEC d'un contrôle ou d'une référence provenant d'un individu sain ou d'une population d'individus sains ; ou
la déviation étant la population de cellules endothéliales circulantes ayant une forme nucléaire anormale par rapport à la forme nucléaire des CEC d'un contrôle ou d'une référence provenant d'un individu sain ou d'une population d'individus sains.

7. Méthode selon l'une quelconque des revendications 2 à 5, la déviation étant la population de cellules endothéliales circulantes ayant une surface cellulaire moyenne qui est supérieure d'au moins 30 % à la surface cellulaire moyenne des CEC d'un contrôle ou d'une référence provenant d'un individu sain ou d'une population d'individus sains.

8. Méthode selon l'une quelconque des revendications 2 à 5, la déviation étant la population de cellules endothéliales circulantes ayant une surface cellulaire moyenne qui est supérieure à environ 100 µm².

9. Méthode selon l'une quelconque des revendications 2 à 5, la déviation étant la population de cellules endothéliales circulantes ayant une surface nucléaire moyenne qui est supérieure d'au moins 30 % à la surface nucléaire moyenne des CEC d'un contrôle ou d'une référence provenant d'un individu sain ou d'une population d'individus sains.

10. Méthode selon l'une quelconque des revendications 2 à 5, la déviation étant la population de cellules endothéliales circulantes ayant une surface nucléaire moyenne qui est supérieure à environ 40 µm².

11. Méthode selon l'une quelconque des revendications 2 à 5, la déviation étant la population de cellules endothéliales circulantes ayant une moyenne d'au moins deux noyaux par CEC.

12. Méthode selon l'une quelconque des revendications 1 à 11, comportant en outre l'étape consistant à comparer :
(a) la concentration de la population de cellules endothéliales circulantes isolées de l'échantillon de sang ; et
(b) la concentration d'un contrôle ou d'une référence.

13. Méthode selon l'une quelconque des revendications 1 à 12, la population de cellules endothéliales circulantes étant isolée de l'échantillon de sang en :
(a) mettant en contact l'échantillon de sang avec des anticorps anti-CD146, les anticorps étant magnétiquement marqués ou marquées par fluorescence ; et
(b) isolant les cellules auxquelles les anticorps anti-CD146 se lient spécifiquement, pour générer une population de cellules CD 146+.

14. Méthode selon la revendication 13, comportant en outre l'étape consistant à mettre en contact la population de cellules CD 146+ avec des anticorps anti-CD45, anticorps anti-CD105, anticorps anti-CD34, anticorps anti-CD31, anticorps anti-VEGF2, anticorps anti-CD144, anticorps anti-CD106, DAPI, ou combinaison de ceux-ci marqués par fluorescence.

15. Méthode selon la revendication 13 or 14, comportant en outre les étapes consistant à rejeter une cellule CD146+ qui se lie spécifiquement à un anticorps anti-CD45 et à retenir une cellule CD 146+ qui ne se lie pas spécifiquement à un anticorps anti-CD45, pour générer une population de cellules endothéliales circulantes isolées.

16. Méthode selon l'une quelconque des revendications 1 à 15, le trouble cardiovasculaire étant choisi parmi : rupture de plaque, érosion de plaque, ischémie du coeur, lésion de reperfusion du coeur, athérosclérose, syndrome coronarien aigu, infarctus du myocarde avec un sus-décalage important du segment ST (STEMI), infarctus du myocarde sans sus-décalage du segment ST (NSTEMI), angine instable, accident ischémique cérébral ou une combinaison de ceux-ci.
